# EUROPEAN PATENT APPLICATION

(11) **EP 4 790 139 A1**
(43) Date of publication of application: **12.08.2026**
(21) Application number: 24874657.0
(22) Date of filing: 02.10.2024
(51) Int. Cl.: C12N 15/11, C07K 4/00, C07K 14/00, C07K 16/00, C12N 1/15, C12N 1/19, C12N 1/21, C12N 5/10, C12N 15/13, C12N 15/63

(54) **METHOD FOR INHIBITING FORMATION OF AGGREGATES OF POLYPEPTIDE**

(30) Priority: 02.10.2023 JP 2023171543
(71) Applicant: National University Corporation Kumamoto University, Kumamoto 860-8555 (JP)
(72) Inventor: MORIOKA, Hiroshi, Kumamoto-shi, Kumamoto 862-0973 (JP); KOBASHIGAWA, Yoshihiro, Kumamoto-shi, Kumamoto 862-0973 (JP); SATO, Takashi, Kumamoto-shi, Kumamoto 862-0973 (JP); OKAZAKI, Takumi, Kumamoto-shi, Kumamoto 862-0973 (JP)
(74) Representative: Plasseraud IP
(86) International application number: PCT/JP2024/035342
(87) International publication number: WO 2025/075057

(57) **Abstract**

The present invention provides a polypeptide comprising a heavy chain variable region (VH) domain and a light chain variable region (VL) domain that are linked via a peptide linker, in which the peptide linker comprises an amino acid sequence of SEQ ID NO: 1 or SEQ ID NO: 2.

## Description

### TECHNICAL FIELD

The present invention relates to a method for inhibiting formation of an aggregate of polypeptides, particularly a single-chain antibody. The present invention further relates to a single-chain antibody in which formation of an aggregate is inhibited, and a method for producing such a single-chain antibody.

### BACKGROUND ART

Monoclonal antibodies are used as therapeutic agents in various clinical settings, including oncology, chronic inflammatory diseases, transplantation, infectious diseases, cardiovascular diseases and ophthalmic diseases, and are also used in various applications such as diagnostic reagents and sensor elements. A primary function of an antibody is to specifically bind to an antigen (target molecule), and the antigen is recognized by an Fv domain.

The Fv domain of an antibody consists of an Fv domain derived from a heavy chain (VH) and an Fv domain derived from a light chain (VL). Many antibodies retain their target binding ability even in Fv fragments, which are fragments obtained by isolating the Fv domain from the antibodies, and thus constitute the minimal unit of the antigen binding function of the antibodies. A single-chain antibody (scFv: single-chain Fv) is obtained by linking the VH and the VL via a peptide linker. The VH and the VL contained in the single-chain antibody associate with each other to form an antigen binding site.

Stability of an antibody molecule is evaluated from three viewpoints. The first viewpoint is thermal resistance or thermal stability. The second viewpoint is stability against a proteolytic enzyme. The third viewpoint is storage stability due to inhibition of formation of an aggregate. With regard to the storage stability, it has been reported that intermolecular association is inhibited and an amount of formed aggregates is reduced by cyclizing a single-chain antibody (PTL 1). Further, there has been a report on a design of a thermostable mutant of a single-chain antibody (NPL 9).

A peptide linker is a synthetic amino acid sequence commonly used to physically connect polypeptide domains. Most peptide linkers are composed of one or more repeating modules of amino acids glycine and serine. For example, a single-chain antibody is known in which a peptide linker consisting of 15 amino acids (GGGGS)₃ is used, and it is considered that such a peptide linker does not interfere with the assembly and binding activity of the linked domains (PTL 2 and NPL 1).

An scFv generally has a property of associating to form a multimer, and there have been reports on a dimer, a trimer, and a tetramer formed by the scFv (NPLs 2 to 4). It is known that, when the peptide linker of a single-chain antibody is so short that the VH and the VL cannot associate with each other in the molecule, the VH and the VL intermolecularly associate with each other to form a dimer (NPL 8). There have been several reports on a peptide linker usable for a single-chain antibody (PTL 2 and NPLs 5 to 7, 9, and 10).

In general, a monoclonal antibody is produced using a eukaryotic cell such as a CHO cell or a hybridoma, and requires enormous production costs. An scFv, however, has a molecular weight of about 25 kDa, and a diabody has a molecular weight of about 50 kDa, and their molecular weights are remarkably smaller than that of a full-length antibody. Therefore, the scFv and the diabody can be produced using a prokaryotic host such as *Escherichia coli* (NPL 7), and they have advantages over full-length antibodies in terms of the production cost.

### CITATION LIST

### PATENT LITERATURE

PTL 1: WO 2020/013126 A1
PTL 2: WO 2012/088461 A2
PTL 3: WO 2006/116657 A2

### NON PATENT LITERATURE

NPL 1: Freund, C. et al., FEBS Letters 320 (1993) 1873-3468;
NPL 2: PEI, X.Y., et al., Proc. Natl. Acad. Sci. USA, 1997, 94, 9637-9642;
NPL 3: Todorovska, A. et al., Journal of Immunological Methods 248 (2001) 47-66;
NPL 4: Lawrence, L.J. et al., FEBS Letters 425 (1998) 479-484;
NPL 5: Arslan, M. et al., Scientific Reports, Volume 12, Article number 5449 (2022);
NPL 6: Tang, Y. et al., J. Biol. Chem., 1996, 271(26), 15682-6;
NPL 7: Liu, C. et al., Journal of Biochemistry, 166, 6, 2019, 455-462;
NPL 8: Holliger, P. et al., Proc. Natl. Acad. Sci. USA, 90, 6444-6448, 1993;
NPL 9: Okazaki, K. et al., ACS Omega 2023, 8, 22945-22954;
NPL 10: Klement, M. et al., Journal of Biotechnology 199 (2015) 90-97

### SUMMARY OF INVENTION

### TECHNICAL PROBLEM

Polypeptides such as an scFv and a diabody are also referred to as low molecular weight antibodies, and have characteristics in terms of permeability to a tissue, ease of production, use in conjugation or the like, the possibility of obtaining an antagonist antibody, and the like, and have been widely studied as a pharmaceutical product. On the other hand, a low molecular weight antibody having properties preferable as an active ingredient of a pharmaceutical product, such as high stability and high binding activity, is required.

### SOLUTION TO PROBLEM

The present inventors have found that a polypeptide designed to have a specific sequence, particularly an scFv, has properties preferable as an active ingredient of a pharmaceutical product, such as the high stability and the high binding activity, and thus completed the present invention.

The present invention provides the following inventions.
[1-1] A polypeptide comprising a heavy chain variable region (VH) domain and a light chain variable region (VL) domain that are linked via a peptide linker,
   wherein the peptide linker comprises an amino acid sequence:
   X¹²X¹³X¹⁴GX¹⁵GQX¹⁶WX¹⁷X¹⁸ (SEQ ID NO: 1); or
   AX³⁴X³⁵X³⁶SGX³⁷X³⁸X³⁹X⁴⁰X⁴¹X⁴²T (SEQ ID NO: 2), and
   wherein each of X¹² to X¹⁸ and X³⁴ to X⁴² is independently selected from naturally occurring amino acid residues.
[1-2] The polypeptide according to [1-1], wherein the peptide linker comprises an amino acid sequence:
   TAGAGQSWYG (SEQ ID NO: 3); or
   AAGGSGSGADGAT (SEQ ID NO: 4).
[1-3] The polypeptide according to [1-1], wherein the peptide linker comprises an amino acid sequence:
   X¹PX²X³X⁴X⁵X⁶X⁷X⁸X⁹X¹⁰X¹¹GX¹²X¹³X¹⁴GX¹⁵GQX¹⁶WX¹⁷X¹⁸ (SEQ ID NO: 5); or
   X³¹X³²X³³AX³⁴X³⁵X³⁶SGX³⁷X³⁸X³⁹X⁴⁰X⁴¹X⁴²TX⁴³X⁴⁴X⁴⁵X⁴⁶X⁴⁷X⁴⁸ (SEQ ID NO: 6), and
   wherein each of X¹ to X¹⁸ and X³¹ to X⁴⁸ is independently selected from naturally occurring amino acid residues.
[1-4] The polypeptide according to [1-1], wherein the peptide linker comprises an amino acid sequence:
   X¹PX²X³X⁴X⁵X⁶X⁷X⁸X⁹X¹⁰X¹¹GATAGAGQSWYG (SEQ ID NO: 7); or
   X³¹X³²X³³AAGGSGSGADGATX⁴³X⁴⁴X⁴⁵X⁴⁶X⁴⁷X⁴⁸ (SEQ ID NO: 8), and
   wherein each of X¹ to X¹¹, X³¹ to X³³, and X⁴³ to X⁴⁸ is independently selected from naturally occurring amino acid residues.
[1-5] The polypeptide according to [1-1], wherein the peptide linker comprises a sequence consisting of 15 or more consecutive amino acids contained in the amino acid sequence:
   GPEEQYSAYATTGATAGAGQSWYG (SEQ ID NO: 9); or
   PGAAAGGSGSGADGATYTTTPG (SEQ ID NO: 10).
[1-6] The polypeptide according to [1-1] or [1-2], wherein the peptide linker comprises a sequence consisting of 15 or more consecutive amino acids contained in the amino acid sequence:
   GGGGSGGGGSGGGSTAGAGQSWYG (SEQ ID NO: 47).
[1-7] The polypeptide according to any one of [1-1] to [1-6], wherein the peptide linker comprises an amino acid sequence:
   GGGGS (SEQ ID NO: 48); or
   GGGS (SEQ ID NO: 49).
[1-8] The polypeptide according to any one of [1-1] to [1-7], wherein the peptide linker comprises an amino acid sequence:
   GGGGSGGGGSGGGS (SEQ ID NO: 50).
[1-9] The polypeptide according to any one of [1-1] to [1-8], wherein the peptide linker consists of from 15 to 60 amino acids.
[1-10] The polypeptide according to [1-8], wherein the peptide linker comprises a sequence consisting of 20 or more consecutive amino acids contained in the amino acid sequence of SEQ ID NO: 9, SEQ ID NO: 10, or SEQ ID NO: 47.
[1-11] The polypeptide according to any one of [1-1] to [1-10], wherein the peptide linker comprises the amino acid sequence of SEQ ID NO: 9, SEQ ID NO: 10, or SEQ ID NO: 47.
[1-12] The polypeptide according to any one of [1-1] to [1-11], wherein the peptide linker comprises the amino acid sequence of SEQ ID NO: 1, and is linked to a C terminus of the VH domain and an N terminus of the VL domain.
[1-13] The polypeptide according to any one of [1-1] to [1-11], wherein the peptide linker comprises the amino acid sequence of SEQ ID NO: 2, and is linked to the C terminus of the VL domain and the N terminus of the VH domain.
[1-14] The polypeptide according to [1-12], wherein the peptide linker consists of from 15 to 30 amino acids.
[1-15] The polypeptide according to [1-13], wherein the peptide linker consists of from 35 to 60 amino acids.
[1-16] The polypeptide according to any one of [1-1] to [1-15], wherein formation of an aggregate is inhibited as compared with a single-chain antibody having the same VH domain and the same VL domain, and having a peptide linker of (G₄S)₃.
[1-17] The polypeptide according to any one of [1-1] to [1-16], wherein the polypeptide is a single-chain antibody (scFv).
[1-18] The polypeptide according to [1-12], wherein the VH domain and the VL domain have the following amino acid residues specified by the Kabat numbering: VL38 (Q), 40 (P), 41 (G), 85 (T), 87 (Y), 95 (P), 97 (T), 98 (F), and 101 (G); and VH41 (P), 42 (G), 46 (E), 62 (S), and 91 (Y).
[1-19] The polypeptide according to [1-13], wherein the VH domain and the VL domain have the following amino acid residues specified by the Kabat numbering: VL38 (Q), 40 (P), 41 (G), 445 (K), 85 (T), 87 (Y), 98 (F), 99 (G), and 101 (G); and VH2 (V), 4 (L), 41 (P), 42 (G), 43 (K), 46 (E), 91 (Y), 102 (Y), 104 (G), 105 (Q), and 106 (G).
[2-1] A polypeptide comprising a heavy chain variable region (VH) domain and a light chain variable region (VL) domain that are linked via a linker, wherein the polypeptide has a peptide tag at an N terminus, the peptide tag including an amino acid sequence:
   X¹²X¹³X¹⁴GX¹⁵GQX¹⁶WX¹⁷X¹⁸ (SEQ ID NO: 1); or
   AX³⁴X³⁵X³⁶SGX³⁷X³⁸X³⁹X⁴⁰X⁴¹X⁴²T (SEQ ID NO: 2), and
   wherein each of X¹² to X¹⁸ and X³⁴ to X⁴² is independently selected from naturally occurring amino acid residues.
[2-2] The polypeptide according to [2-1], wherein the peptide tag comprises an amino acid sequence:
   TAGAGQSWYG (SEQ ID NO: 3); or
   AAGGSGSGADGAT (SEQ ID NO: 4).
[2-3] The polypeptide according to [2-1], wherein the peptide tag comprises an amino acid sequence:
   X¹PX²X³X⁴X⁵X⁶X⁷X⁸X⁹X¹⁰X¹¹GX¹²X¹³X¹⁴GX¹⁵GQX¹⁶WX¹⁷X¹⁸ (SEQ ID NO: 5); or
   X³¹X³²X³³AX³⁴X³⁵X³⁶SGX³⁷X³⁸X³⁹X⁴⁰X⁴¹X⁴²TX⁴³X⁴⁴X⁴⁵X⁴⁶X⁴⁷X⁴⁸ (SEQ ID NO: 6), and
   wherein each of X¹ to X¹⁸ and X³¹ to X⁴⁸ is independently selected from naturally occurring amino acid residues.
[2-4] The polypeptide according to [2-1], wherein the peptide tag comprises an amino acid sequence:
   X¹PX²X³X⁴X⁵X⁶X⁷X⁸X⁹X¹⁰X¹¹GATAGAGQSWYG (SEQ ID NO: 7); or
   X³¹X³²X³³AAGGSGSGADGATX⁴³X⁴⁴X⁴⁵X⁴⁶X⁴⁷X⁴⁸ (SEQ ID NO: 8), and
   wherein each of X¹ to X¹¹, X³¹ to X³³, and X⁴³ to X⁴⁸ is independently selected from naturally occurring amino acid residues.
[2-5] The polypeptide according to [2-4], wherein the peptide tag comprises a sequence consisting of 15 or more consecutive amino acids contained in the amino acid sequence:
   GPEEQYSAYATTGATAGAGQSWYG (SEQ ID NO: 9); or
   PGAAAGGSGSGADGATYTTTPG (SEQ ID NO: 10).
[2-6] The polypeptide according to [2-1] or [2-2], wherein the peptide tag comprises a sequence consisting of 15 or more consecutive amino acids contained in the amino acid sequence:
   GGGGSGGGGSGGGSTAGAGQSWYG (SEQ ID NO: 47).
[2-7] The polypeptide according to any one of [2-1] to [2-6], wherein the peptide tag comprises an amino acid sequence:
   GGGGS (SEQ ID NO: 48); or
   GGGS (SEQ ID NO: 49).
[2-8] The polypeptide according to any one of [2-1] to [2-7], wherein the peptide tag comprises an amino acid sequence:
   GGGGSGGGGSGGGS (SEQ ID NO: 50).
[2-9] The polypeptide according to [2-5] or [2-6], wherein the peptide tag consists of from 15 to 60 amino acids.
[2-10] The polypeptide according to [2-5], wherein the peptide tag comprises a sequence consisting of 20 or more consecutive amino acids contained in the amino acid sequence of SEQ ID NO: 9 or SEQ ID NO: 10.
[2-11] The polypeptide according to any one of [2-1] to [2-10], wherein the peptide tag comprises the amino acid sequence of SEQ ID NO: 9 or SEQ ID NO: 10.
[2-12] The polypeptide according to any one of [2-1] to [2-11], wherein the peptide tag comprises the amino acid sequence of SEQ ID NO: 1, wherein the linker is linked to a C terminus of the VH domain and an N terminus of the VL domain, and wherein the peptide tag is linked to a C terminus of the VL domain.
[2-13] The polypeptide according to any one of [2-1] to [2-11], wherein the peptide tag comprises the amino acid sequence of SEQ ID NO: 2, wherein the linker is linked to a C terminus of the VL domain and an N terminus of the VH domain, and wherein the peptide tag is linked to a C terminus of the VL domain.
[2-14] The polypeptide according to [2-12], wherein the peptide tag consists of from 15 to 30 amino acids.
[2-15] The polypeptide according to [2-10], wherein the peptide tag consists of from 35 to 60 amino acids.
[2-16] The polypeptide according to any one of [2-1] to [2-15], wherein formation of an aggregate is inhibited as compared with a polypeptide having the same linker, the same VH domain, and the same VL domain, but lacking a peptide tag.
[2-17] The polypeptide according to any one of [2-1] to [2-16], wherein the linker is a peptide linker.
[2-18] The polypeptide according to any one of [2-1] to [2-17], wherein the polypeptide is a single-chain antibody (scFv).
[2-19] The polypeptide according to [2-12], wherein the VH domain and the VL domain have the following amino acid residues specified by the Kabat numbering: VL38 (Q), 40 (P), 41 (G), 85 (T), 87 (Y), 95 (P), 97 (T), 98 (F), and 101 (G); and VH41 (P), 42 (G), 46 (E), 62 (S), and 91 (Y).
[2-20] The polypeptide according to [2-13], wherein the VH domain and the VL domain have the following amino acid residues specified by the Kabat numbering: VL38 (Q), 40 (P), 41 (G), 45 (K), 85 (T), 87 (Y), 98 (F), 99 (G), and 101 (G); and VH2 (V), 4 (L), 41 (P), 42 (G), 43 (K), 46 (E), 91 (Y), 102 (Y), 104 (G), 105 (Q), and 106 (G).
[3-1] A nucleic acid including a nucleotide sequence that encodes the amino acid sequence of the polypeptide according to any one of [1-1] to [1-19] and [2-1] to [2-20].
[3-2] A recombinant vector including the nucleic acid according to [3-1].
[3-3] A transformant into which the recombinant vector according to [3-2] has been introduced.

### ADVANTAGEOUS EFFECTS OF INVENTION

According to the present invention, an scFv is provided which has high binding activity to an antigen and high stability, particularly an scFv in which intermolecular formation of an aggregate is inhibited. Such an scFv can be used as a sensor element for analysis or diagnosis, as a pharmaceutical product, and the like.

### BRIEF DESCRIPTION OF DRAWINGS

[Fig. 1] Fig. 1 shows results of purification of an scFv (Tras-scFv-HL-Glue) by gel filtration chromatography and results of SDS-PAGE.
[Fig. 2] Fig. 2 shows results of purification of an scFv (Tras-scFv-HL) by gel filtration chromatography and results of SDS-PAGE.
[Fig. 3] Fig. 3 shows a comparison of the results of purification by gel filtration chromatography between a control and the scFvs (Tras-scFv-HL and Tras-scFv-HL-Glue).
[Fig. 4] Fig. 4 shows a comparison of the results of analysis by gel filtration chromatography between the scFvs (Tras-scFv-HL and Tras-scFv-HL-Glue).
[Fig. 5] Fig. 5 is a graph showing results of evaluation of aggregation stability of the scFv (Tras-scFv-HL-Glue) by dynamic light scattering.
[Fig. 6] Fig. 6 is a graph showing results of measurement by surface plasmon resonance for the scFv (Tras-scFv-HL-Glue).
[Fig. 7] Fig. 7 shows results of purification of an scFv (Y9-scFv-HL) by gel filtration chromatography and results of SDS-PAGE.
[Fig. 8] Fig. 8 shows results of purification of an scFv (Y9-scFv-HL-Glue) by gel filtration chromatography and results of SDS-PAGE.
[Fig. 9] Fig. 9 shows a comparison of the results of analysis by gel filtration chromatography between a control and the scFv (Y9-scFv-HL-Glue).
[Fig. 10] Fig. 10 is a graph showing results of evaluation of aggregation stability of the scFv (Y9-scFv-HL-Glue) by dynamic light scattering.
[Fig. 11] Fig. 11 shows a structural model of the scFv (Tras-scFv-HL-Glue) obtained by molecular dynamics calculations.

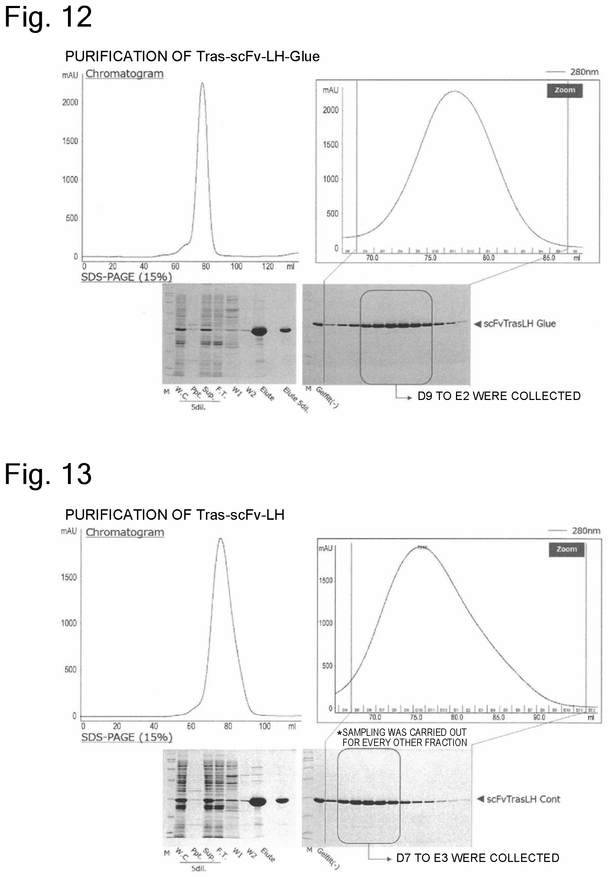

[Fig. 12] Fig. 12 shows results of purification of an scFv (Tras-scFv-LH-Glue) by gel filtration chromatography and results of SDS-PAGE.
[Fig. 13] Fig. 13 shows results of purification of an scFv (Tras-scFv-LH) by gel filtration chromatography and results of SDS-PAGE.

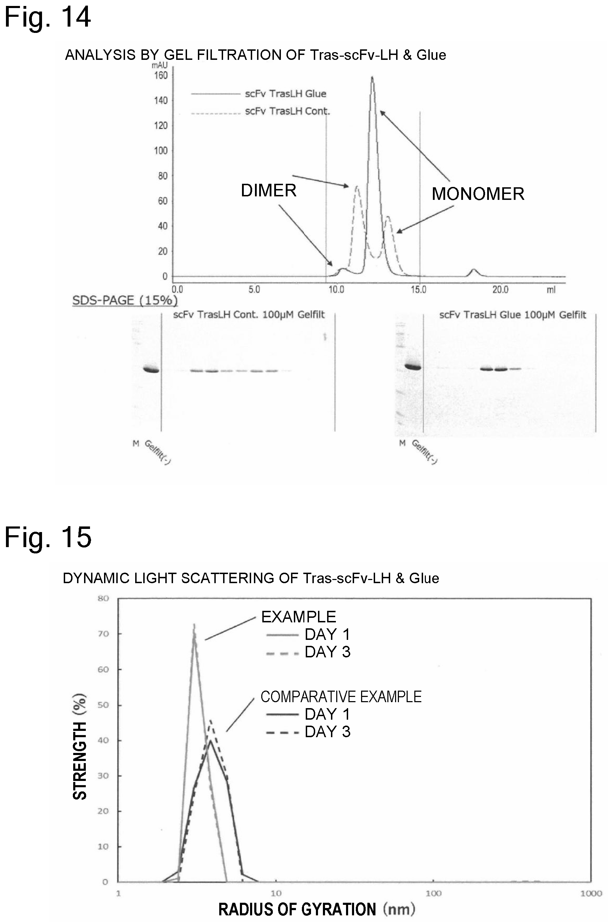

[Fig. 14] Fig. 14 shows a comparison of the results of analysis by gel filtration chromatography between the scFvs (Tras-scFv-LH and Tras-scFv-LH-Glue).
[Fig. 15] Fig. 15 is a graph showing results of evaluation of aggregation stability of the scFvs (Tras-scFv-LH and Tras-scFv-LH-Glue) by dynamic light scattering.
[Fig. 16] Fig. 16 shows results of evaluation of antigen binding activity of the T scFvs (Tras-scFv-LH and Tras-scFv-LH-Glue) by a pull-down assay.
[Fig. 17] Fig. 17 shows results of purification of an scFv (Y9-scFv-LH-Glue) by gel filtration chromatography and results of SDS-PAGE.

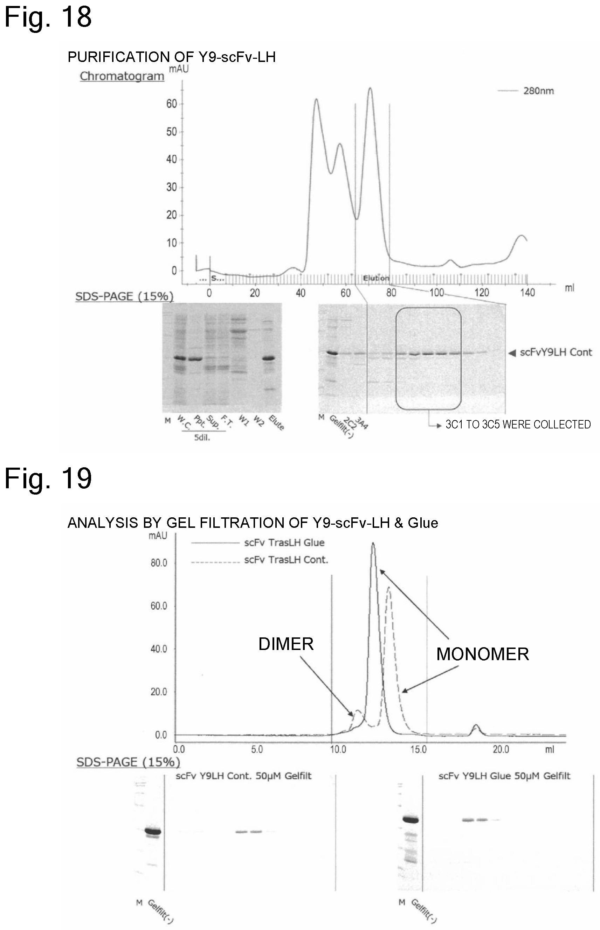

[Fig. 18] Fig. 18 shows results of purification of an scFv (Y9-scFv-LH) by gel filtration chromatography and results of SDS-PAGE.
[Fig. 19] Fig. 19 shows a comparison of the results of analysis by gel filtration chromatography between the scFvs (Y9-scFv-LH and Y9-scFv-LH-Glue).
[Fig. 20] Fig. 20 is a graph showing results of evaluation of aggregation stability of the scFvs (Y9-scFv-LH and Y9-scFv-LH-Glue) by dynamic light scattering.
[Fig. 21] Fig. 21 shows a structural model of the scFv (Tras-scFv-LH-Glue) obtained by molecular dynamics calculations.
[Fig. 22] Fig. 22 is a structural model diagram showing a binding surface of a core sequence of the HL-Glue in the scFv (Tras-scFv-HL-Glue). The binding surface of the core sequence of the HL-Glue is shown in black, the VH is shown in light gray, and the VL is shown in dark gray.
[Fig. 23] Fig. 23 is a structural model diagram showing a binding surface of a core sequence of the LH-Glue in the scFv (Tras-scFv-LH-Glue) obtained by molecular dynamics calculations. The binding surface of the core sequence of the LH-Glue is shown in black, the VH is shown in light gray, and the VL is shown in dark gray.
[Fig. 24] Fig. 24 shows a comparison of the results of analysis by gel filtration chromatography between scFvs (Tras-scFv-LH and GlueCore-Tras-scFv-LH).
[Fig. 25] Fig. 25 shows results of analysis by gel filtration chromatography for an scFv (Tras-scFv-HL-Glue2) prepared in Example 6.
[Fig. 26] Fig. 26 shows results of SDS-PAGE after gel filtration chromatography purification for the scFv (Tras-scFv-HL-Glue2) prepared in Example 6.
[Fig. 27] Fig. 27 shows a comparison of the results of purification by gel filtration chromatography between a control and the scFvs (Tras-scFv-HL and Tras-scFv-HL-Glue2).

### DESCRIPTION OF EMBODIMENTS

In one aspect of the present invention, there is provided a polypeptide including a peptide linker having a specific sequence, in which a VH domain and a VL domain contained in the polypeptide associate with each other in the molecule and have binding activity to an antigen.

In one aspect of the present invention, the VH domain and the VL domain are a VH and a VL each composed of about 100 to 120 amino acid residues referred to as an "immunoglobulin fold," have the same overall conformation, and are mainly composed of β-sheets. In one aspect of the present invention, the scFv may be arranged either in the order of the VH domain and the VL domain when seen from the N terminus, or in the order of the VL domain and the VH domain when seen from the N terminus. In one aspect of the present invention, the VH domain and the VL domain are each the VH and the VL, or functional fragments thereof.

In an antibody, a region corresponding to each of a VH domain and a VL domain can be determined based on known knowledge. For example, reference may be made to a literature such as Martin, A. C. R., Accessing the Kabat Antibody Sequence Database by Computer, PROTEINS: Structure, Function and Genetics, 25 (1996), 130-133; and Elvin A. Kabat, Tai Te Wu, Carl Foeller, Harold M. Perry, Kay S. Gottesman (1991) Sequences of Proteins of Immunological Interest. In one aspect of the present invention, the VH region is defined as an amino acid sequence of from 0 to 113 in accordance with the Kabat numbering, and the VL region is defined as an amino acid sequence of from 0 to 109 in accordance with the Kabat numbering. In one embodiment, for both the VH region and the VL region, for an antibody in which the two to three residues on the C terminal side of framework 4 do not form a secondary structure, a region excluding such residues may be defined as the VH or the VL.

In one aspect of the present invention, there is provided a polypeptide including a heavy chain variable region (VH) domain and a light chain variable region (VL) domain that are linked via a peptide linker, wherein the peptide linker comprises a sequence consisting of eight or more, nine or more, 10 or more, 11 or more, 12 or more, 13 or more, 14 or more, 15 or more, 16 or more, 17 or more, 18 or more, 19 or more, or 20 or more consecutive amino acids contained in the amino acid sequence:
GPEEQYSAYATTGATAGAGQSWYGE. In one aspect of the present invention, the peptide linker is a polypeptide consisting of from 15 to 32, from 15 to 31, from 15 to 30, from 15 to 29, from 15 to 28, from 15 to 27, from 15 to 26, or from 15 to 25 amino acids.

In one aspect of the present invention, the peptide linker is a polypeptide including the amino acid sequence: X¹²X¹³X¹⁴GX¹⁵GQX¹⁶WX¹⁷X¹⁸ (SEQ ID NO: 1). In another aspect of the present invention, the peptide linker is a polypeptide including the amino acid sequence: TAGAGQSWYG (SEQ ID NO: 3). In still another aspect of the present invention, the peptide linker is a polypeptide including the amino acid sequence: X¹PX²X³X⁴X⁵X⁶X⁷X⁸X⁹X¹⁰X¹¹GX¹²X¹³X¹⁴GX¹⁵GQX¹⁶WX¹⁷X¹⁸ (SEQ ID NO: 5). In yet another aspect of the present invention, the peptide linker is a polypeptide including the amino acid sequence: X¹PX²X³X⁴X⁵X⁶X⁷X⁸X⁹X¹⁰X¹¹GATAGAGQSWYG (SEQ ID NO: 7).

In one aspect of the present invention, the peptide linker is a polypeptide including the amino acid sequence: TAGAGQSWYG (SEQ ID NO: 3). In one embodiment of this aspect, the peptide linker is a polypeptide consisting of from 15 to 32, from 15 to 31, from 15 to 30, from 15 to 29, from 15 to 28, from 15 to 27, from 15 to 26, or from 15 to 25 amino acids.

In one aspect of the present invention, the peptide linker is a polypeptide including the amino acid sequence: AX³⁴X³⁵X³⁶SGX³⁷X³⁸X³⁹X⁴⁰X⁴¹X⁴²T (SEQ ID NO: 2). In another aspect of the present invention, the peptide linker is a polypeptide including the amino acid sequence: AAGGSGSGADGAT (SEQ ID NO: 4). In another aspect of the present invention, the peptide linker is a polypeptide including the amino acid sequence: X³¹X³²X³³AX³⁴X³⁵X³⁶SGX³⁷X³⁸X³⁹X⁴⁰X⁴¹X⁴²TX⁴³X⁴⁴X⁴⁵X⁴⁶X⁴⁷X⁴⁸ (SEQ ID NO: 6). In still another aspect of the present invention, the peptide linker is a polypeptide including the amino acid sequence: X³¹X³²X³³AAGGSGSGADGATX⁴³X⁴⁴X⁴⁵X⁴⁶X⁴⁷X⁴⁸ (SEQ ID NO: 8).

In one aspect of the present invention, the peptide linker is a polypeptide including the amino acid sequence: AAGGSGSGADGAT (SEQ ID NO: 4). In one embodiment of this aspect, the peptide linker is a polypeptide consisting of from 15 to 60, from 15 to 59, from 15 to 58, from 15 to 57, from 15 to 56, from 15 to 55, from 15 to 54, or from 15 to 53 amino acids.

In one aspect of the present invention, each of X¹ to X¹⁸ is independently selected from naturally occurring amino acid residues, that is, alanine (A), arginine (R), asparagine (N), aspartic acid (D), cysteine (C), glutamine (Q), glutamic acid (E), glycine (G), histidine (H), isoleucine (I), leucine (L), lysine (K), methionine (M), phenylalanine (F), proline (P), serine (S), threonine (T), tryptophan (W), tyrosine (Y), and valine (V). In one aspect of the present invention, each of X¹ to X¹⁸ is independently selected from glycine (G), glutamic acid (E), glutamine (Q), tyrosine (Y), serine (S), alanine (A), and threonine (T).

In one aspect of the present invention, the peptide linker is a polypeptide including a sequence having 80% or more, 85% or more, 90% or more, or 95% or more sequence identity to the amino acid sequence: GPEEQYSAYATTGATAGAGQSWYGE.

In one aspect of the present invention, the amino acids constituting the peptide linker are not particularly limited as long as they are amino acids, and may be either naturally occurring amino acids or non-naturally occurring amino acids. Examples of such amino acids include glycine, alanine, valine, isoleucine, leucine, serine, threonine, cysteine, methionine, phenylalanine, tryptophan, tyrosine, proline, glutamic acid, aspartic acid, glutamine, asparagine, lysine, arginine and histidine, and specific examples include glycine, alanine, serine, phenylalanine, glutamic acid and arginine.

In one embodiment of the present invention, the polypeptide having a peptide linker may have a structure in which the VH domain, the peptide linker, and the VL domain are linked in this order when seen from the N terminus, or may have a structure in which the VL domain, the peptide linker, and the VH domain are linked in this order when seen from the N terminus. In the present specification, a single-chain antibody in which the C terminus of the VH domain is linked to the VL domain via a peptide linker is referred to as an "scFv-HL," and a single-chain antibody in which the C terminus of the VL domain is linked to the VH domain via a peptide linker is referred to as an "scFv-LH."

In one aspect of the present invention, the peptide linker is a polypeptide including the amino acid sequences of SEQ ID NOs: 1 to 4, and has a structure in which the VH domain, the peptide linker, and the VL domain are linked in this order when seen from the N terminus.

In one aspect of the present invention, there is provided a polypeptide including a heavy chain variable region (VH) domain and a light chain variable region (VL) domain that are linked via a linker, and having a peptide tag at the C terminus. Here, the linker is not particularly limited as long as it links the VH domain and the VL domain by a covalent bond, and examples thereof include peptide linkers. In one embodiment, the linker is a peptide linker having any sequence with a length of 12 residues or more, for example, GSGGGGSGGGGSGGGGS.

In one aspect of the present invention, the peptide tag is a polypeptide including the amino acid sequence: X¹²X¹³X¹⁴GX¹⁵GQX¹⁶WX¹⁷X¹⁸ (SEQ ID NO: 1). In another aspect of the present invention, the peptide tag is a polypeptide including the amino acid sequence: TAGAGQSWYG (SEQ ID NO: 3). In still another aspect of the present invention, the peptide tag is a polypeptide including the amino acid sequence: X¹PX²X³X⁴X⁵X⁶X⁷X⁸X⁹X¹⁰X¹¹GX¹²X¹³X¹⁴GX¹⁵GQX¹⁶WX¹⁷X¹⁸ (SEQ ID NO: 5). In yet another aspect of the present invention, the peptide tag is a polypeptide including the amino acid sequence: X¹PX²X³X⁴X⁵X⁶X⁷X⁸X⁹X¹⁰X¹¹GATAGAGQSWYG (SEQ ID NO: 7).

In one aspect of the present invention, the peptide tag is a polypeptide including the amino acid sequence: TAGAGQSWYG (SEQ ID NO: 3). In one embodiment of this aspect, the peptide tag is a polypeptide consisting of from 15 to 32, from 15 to 31, from 15 to 30, from 15 to 29, from 15 to 28, from 15 to 27, from 15 to 26, or from 15 to 25 amino acids.

In one aspect of the present invention, the peptide tag is a polypeptide including the amino acid sequence: AX³⁴X³⁵X³⁶SGX³⁷X³⁸X³⁹X⁴⁰X⁴¹X⁴²T (SEQ ID NO: 2). In another aspect of the present invention, the peptide linker is a polypeptide including the amino acid sequence: AAGGSGSGADGAT (SEQ ID NO: 4). In another aspect of the present invention, the peptide tag is a polypeptide including the amino acid sequence: X³¹X³²X³³AX³⁴X³⁵X³⁶SGX³⁷X³⁸X³⁹X⁴⁰X⁴¹X⁴²TX⁴³X⁴⁴X⁴⁵X⁴⁶X⁴⁷X⁴⁸ (SEQ ID NO: 6). In still another aspect of the present invention, the peptide linker is a polypeptide including the amino acid sequence: X³¹X³²X³³AAGGSGSGADGATX⁴³X⁴⁴X⁴⁵X⁴⁶X⁴⁷X⁴⁸ (SEQ ID NO: 8).

In one aspect of the present invention, the peptide tag is a polypeptide including the amino acid sequence: AAGGSGSGADGAT (SEQ ID NO: 4). In one embodiment of this aspect, the peptide linker is a polypeptide consisting of from 15 to 60, from 15 to 59, from 15 to 58, from 15 to 57, from 15 to 56, from 15 to 55, from 15 to 54, or from 15 to 53 amino acids.

In one embodiment of the present invention, any host including *Escherichia coli*, yeast, mammalian cells and insect cells can be used for production of the polypeptide, and any promoter including T7, Taq and lac can be used as a promoter.

In one embodiment of the present invention, the polypeptide may be fused to another peptide or protein, or a fragment thereof, to enhance the solubility. Examples of peptides and proteins used for the fusion include maltose-binding protein (MBP), thioredoxin, glutathione S-transferase (GST) and protein GB1 domain (GB1).

In one aspect of the present invention, the polypeptide including a peptide linker is a single-chain antibody. As used herein, the term "single-chain antibody (scFv)" means a single-chain Fv fragment (scFv) obtained by linking an Fv domain derived from a heavy chain of a full-length antibody (VH) and an Fv domain derived from a light chain of the full-length antibody (VL) via a peptide linker.

In one aspect of the present invention, the single-chain antibody may have either a structure in which the C terminus of the VL domain and the N terminus of the VH domain are linked via a peptide linker, or a structure in which the C terminus of the VH domain and the N terminus of the VL domain are linked via a peptide linker.

The scFv used in the present invention can be produced by a method described in a standard book (for example, Carl A. K. Borrebaeck (ed.), (1995) Antibody Engineering (Second Edition), Oxford University Press, New York; John McCafferty, Hennie Hoogenboom, Dave Chiswell (eds.), (1996) Antibody Engineering, A Practical Approach, IRL Press, Oxford) and a literature (for example, Biochim. Biophys. Acta - Protein Structure and Molecular Enzymology 1385, 17-32 (1998), Molecules. 2017 22: e1695, and Journal of Biochemistry 161: 37-43).

In one aspect of the present invention, the polypeptides such as an scFv can be produced by culturing a transformant prepared by genetic engineering. A nucleic acid encoding the amino acid sequence of a polypeptide such as a single-chain antibody can be synthesized by chemical synthesis, PCR, cassette mutagenesis, site-directed mutagenesis, and the like. For example, a target nucleic acid can be fully synthesized by chemically synthesizing multiple oligonucleotides of up to about 100 bases, each having a complementary region of about 20 base pairs at the ends, and combining them to perform the overlap extension method.

The recombinant vector of the present invention can be obtained by linking (inserting) the nucleic acid described above into an appropriate vector. The vector used in the present invention is not particularly limited as long as it is capable of replication in a host or capable of integration of the target nucleic acid into the host genome. Examples thereof include bacteriophages, plasmids, cosmids and phagemids.

Examples of the plasmid DNA include plasmids derived from actinomycetes (for example, pK4, pRK401, pRF31, and the like), plasmids derived from *Escherichia coli* (for example, pBR322, pBR325, pUC118, pUC119, pUC18, and the like), plasmids derived from *Bacillus subtilis* (for example, pUB110, pTP5, and the like), and plasmids derived from yeast (for example, YEp13, YEp24, YCp50, and the like). Examples of the phage DNA include λ phages (λgt10, λgt11 and λZAP). In addition, animal virus vectors such as retrovirus or vaccinia virus, and insect virus vectors such as baculovirus can also be used.

In one embodiment of the present invention, the plasmid DNA can be prepared using pNMK (PTL 2 and NPL 7) as a template. In another embodiment of the present invention, a similar construct that can be produced using a vector such as pET28 or pRSF-Duet (both available from Millipore) can be used.

In order to insert a gene into a vector, a method may be employed in which a purified DNA is first digested with an appropriate restriction enzyme, and the gene is inserted into a restriction enzyme site or a multiple cloning site of an appropriate vector DNA to link the gene to the vector. The gene needs to be incorporated into the vector such that the improved protein of the present invention is expressed. Accordingly, the vector of the present invention can be linked with, in addition to a promoter and the nucleotide sequence of the gene, a cis-element such as an enhancer, a splicing signal, a poly(A) addition signal, a selection marker, a ribosome-binding sequence (SD sequence), a start codon, a stop codon, or the like, as desired. In addition, a tag sequence for facilitating purification of the protein to be produced can also be linked. As the tag sequence, a nucleotide sequence encoding a known tag such as a His tag, a GST tag, or an MBP tag can be used.

Whether the gene has been inserted into the vector can be confirmed using known genetic engineering techniques. For example, in the case of a plasmid vector, the vector can be subcloned using a competent cell, and after the DNA is extracted, confirmation can be made by determining the nucleotide sequence using a DNA sequencer. The same method can be used for other vectors that can be subcloned using bacteria or other hosts. Further, selection of vectors using a selection marker such as a drug resistance gene is also effective.

A transformant can be obtained by introducing the recombinant vector of the present invention into a host cell such that the improved protein of the present invention can be expressed. The host used for the transformation is not particularly limited as long as it can express a protein or a polypeptide. Examples thereof include bacteria (such as *Escherichia coli* and *Bacillus subtilis*)*,* yeast, plant cells, animal cells (such as COS cells and CHO cells), and insect cells.

When bacteria are used as the host, it is preferable that the recombinant vector is capable of autonomous replication in the bacteria, and at the same time is composed of a promoter, a ribosome-binding sequence, a start codon, a nucleic acid encoding the improved protein of the present invention, and a transcription termination sequence. Examples of *Escherichia coli* include *Escherichia coli* DH5α, and examples of *Bacillus subtilis* include *Bacillus subtilis.* The method for introducing the recombinant vector into the bacteria is not particularly limited as long as it is a method for introducing a DNA into bacteria. Examples of such a method include a method using calcium ions and electroporation.

When the polypeptide is synthesized in a cell, a peptide involved in folding of the peptide may be co-expressed. Examples of the peptide to be co-expressed include sulfhydryl oxidase (for example, Erv1p), disulfide isomerase (for example, DsbC, PDI, and the like), a MarR-like regulatory factor (for example, SlyA), TF (a trigger factor), and peptidyl prolyl isomerase (PPIase) (for example, cyclophilin, FKBP, Pin1, and the like; specifically, FKBP12).

In one aspect of the present invention, a polypeptide having a peptide linker has, for example, a molecular weight of the scFv of from 10,000 to 60,000 Da, specifically from 15,000 to 50,000 Da, more specifically from 20,000 to 40,000 Da, and still more specifically from 25,000 to 30,000 Da. In one aspect of the present invention, the molecular weight of the scFv is, for example, from 10,000 to 60,000 Da, specifically from 15,000 to 50,000 Da, more specifically from 20,000 to 40,000 Da, and still more specifically from 25,000 to 30,000 Da.

In one aspect of the present invention, the antigen binding activity of a polypeptide such as an scFv is represented by an EC50 value (that is, the 50% binding concentration). The EC50 value can be calculated, for example, based on a measurement result obtained by a method known in the art, such as ELISA, Western blotting, and FACS. In one aspect of the present invention, the antigen binding activity of a polypeptide such as an scFv is represented by a dissociation rate constant (kd), a dissociation constant (Kd), and the like. These can be calculated, for example, based on a measurement result obtained by a known method using surface plasmon resonance.

Hereinafter, the present invention will be illustrated by way of Examples, but the present invention is not intended to be limited thereto.

### [Comparative Example 1] Production of an scFv (Tras-scFv-HL) derived from trastuzumab

An scFv (Tras-scFv-HL) as a comparative example was prepared by the method described in NPL 9 (ACS Omega 2023, 8, 22945-22954). A coding sequence of trastuzumab VH-(GGGS)₃-VL (Tras-scFv-HL, SEQ ID NO: 11) was synthesized using codons optimized for *E. coli* (available from Integrated DNA Technologies) (SEQ ID NO: 12, PTL 1), and was cloned into the pET-28b(+) plasmid using Nco I and Not I restriction enzyme sites. A hexahistidine tag was added following the Tras-scFv, and the Tras-scFv was purified by affinity chromatography using the Ni-NTA agarose resin (available from WAKO). The obtained plasmid was named pET28-Tras-scFv.
The amino acid sequence of the Tras-scFv-HL (SEQ ID NO: 11) 
MEVQLVESGGGLVQPGGSLRLSCAASGFNIKDTYIHWVRQAPGKGLEWVA
RIYPTNGYTRYADSVKGRFTISADTSKNTAYLQMNSLRAEDTAVYYCSRWGGDGFY
AMDYWGQGTLVTVSSGGGGSGGGGSGGGGSDIQMTQSPSSLSASVGDRVTITCRAS
QDVNTAVAWYQQKPGKAPKLLIYSASFLYSGVPSRFSGSRSGTDFTLTISSLQPEDFA
 
TYYCQQHYTTPPTFGQGTKVEIKSAAALEHHHHHH (GGGGSGGGGSGGGGS is the
peptide linker).
The nucleic acid sequence encoding the Tras-scFv-HL (SEQ ID NO: 12)

atggaagtgcagctggttgaaagcggtggcggtctggtgcaaccgggcggtagcctgcgtctgagctgcgcggcgag
cggttttaacatcaaagacacctacattcactgggttcgtcaagcgccgggcaagggtctggagtgggttgcgcgtatctatccgacca
acggctacacccgttatgcggacagcgtgaaaggtcgttttaccattagcgcggataccagcaagaacaccgcgtacctgcagatga
acagcctgcgtgcggaagacaccgcggtttactattgcagccgttggggcggtgacggcttctacgcgatggattattggggccaag
gcaccctggtgaccgttagcagcggcggtggaggtagtggtggaggttccggtggtggcggtagcgatatccagatgacccag
agcccgagcagcctgagcgcgagcgtgggtgaccgtgttaccattacctgccgtgcgagccaagatgtgaacaccgcggttgcgtg
gtatcagcaaaagccgggcaaagcgccgaagctgctgatctacagcgcgagcttcctgtatagcggtgtgccgagccgttttagcgg
cagccgtagcggcaccgacttcaccctgaccattagcagcctgcagccggaggattttgcgacctactattgccagcaacactatacc
accccgccgacctttggtcaaggcaccaaagttgaaatcaagagcgcggccgcactcgagcaccaccaccaccaccactga.

A nucleic acid sequence encoding Erv1p (SEQ ID NO: 13) was synthesized using codons optimized for *E. coli*, and cloned into the first multiple cloning site of the plasmid pCDF-Duet-1. This plasmid was named pCDF-Erv1p.
The nucleic acid sequence encoding the Erv1p (SEQ ID NO: 13) 
atgaaagcaatagataaaatgacggataatccaccacaagaaggcttaagtgggaggaaaataatatatgacgaagatg
gcaaaccttgccgatcatgtaacaccctacttgactttcagtacgtgaccgggaagatatctaatggcctgaagaacctctcatctaacg
gtaaactagcaggtacgggggctctcactggcgaagcttcagagttgatgcctggctcaagaacatacaggaaggttgaccctcctga
cgtagagcaactaggtagatcttcatggacgctgttacactctgtagctgccagctatcctgctcaacctacagaccaacagaagggtg
aaatgaaacagttcttgaatatcttctcacatatttatccttgcaactggtgtgctaaagactttgaaaaatatatcagagaaaatgcaccac
aagttgagtcaagagaagaacttgggaggtggatgtgtgaagcccacaataaagtcaataagaaattgaggaagcccaaatttgactg
taatttctgggaaaaaagatggaaggacggctgggacgaataa.
The amino acid sequence of the Erv1p (SEQ ID NO: 14)

MKAIDKMTDNPPQEGLSGRKIIYDEDGKPCRSCNTLLDFQYVTGKISNGLKN
LSSNGKLAGTGALTGEASELMPGSRTYRKVDPPDVEQLGRSSWTLLHSVAASYPAQ
PTDQQKGEMKQFLNIFSHIYPCNWCAKDFEKYIRENAPQVESREELGRWMCEAHNK
VNKKLRKPKFDCNFWEKRWKDGWDE.

The coding sequence of the Erv1p was amplified from the plasmid pCDF-Erv1p using primers Erv1p-Rv (5'-cgagatctcgatctcctttgttcgacttaagcattattcgtcc-3'; SEQ ID NO: 15) and Erv1p-Fw (5'-ggccacgatgcgtccctgacctcctatgcactcctgc-3'; SEQ ID NO: 16). A vector fragment of pET21-DsbC (see JP 2022-178934 A) was amplified using primers pET21DsbC-Fw (5'-gaggatcgagatctcgatccgcgaaattaatacgactcac-3'; SEQ ID NO: 17) and pET21DsbC-Rv (5'-ggacgcatcgtggccggcatcaccggcc-3'; SEQ ID NO: 18). The two fragments thus obtained were linked using NEBuilder HiFi DNA assembly (available from NEB), and the resulting plasmid was named pET21-Erv1p-DsbC. The plasmid pET21-Erv1p-DsbC was used to express both the Erv1p and the DsbC proteins in the cytoplasm.

SHuffle T7 *E. coli* (available from NEB) was co-transformed with the pET28-Tras-scFv and the pET21-Erv1p-DsbC.

Transformants were selected by spreading on LB plates containing ampicillin (100 mg/L), kanamycin (50 mg/L), and streptomycin (50 mg/L). The colony was inoculated into 50 mL of a 2x YT culture medium containing ampicillin (100 mg/L), kanamycin (50 mg/L), and streptomycin (50 mg/L), and cultured overnight at 30°C. Cells were collected by centrifugal separation (at 6,000 rpm, for 10 min., at 25°C), resuspended in 300 mL of the 2x YT culture medium containing ampicillin (100 mg/L), kanamycin (50 mg/L), and streptomycin (50 mg/L), and stirred at 30°C until the optical density at 600 nm reached 2.5 to 3.0. The Tras-scFv, the DsbC, and the Erv1p was induced with 1 mM IPTG. After stirring the culture solution at 15°C for 48 hours or more, cells were collected by centrifugal separation (at 6,000 rpm, for 10 min., at 4°C), washed with 30 mL of physiological saline, and collected again by centrifugal separation (at 6,000 rpm, for 20 min, at 4°C). The collected cells were suspended in a buffer containing 50 mM Tris-HCl (with a pH value of 8.0) and 150 mM NaCl, and disrupted by sonication on ice. After centrifugal separation (at 12,000 rpm, for 40 min., at 4°C), the supernatant was collected and loaded onto a column packed with Ni-NTA agarose (available from FUJIFILM Wako Pure Chemical). The column was washed with 10 bed volumes (20 mL) of 50 mM Tris-HCl (with a pH value of 8.0) containing both 500 mM NaCl and 20 mM imidazole. The scFv protein was eluted with a solution of 50 mM Tris-HCl (with a pH value of 8.0), 300 mM NaCl, and 250 mM imidazole. The eluate was collected and subjected to centrifugal separation (at 12,000 rpm, for 10 min., at 4°C), and then purified by gel filtration chromatography using a HiLoad 16/600 Superdex 75 pg (prep grade) column (available from GE Healthcare) with a running buffer of 50 mM HEPES (with a pH value of 7.4) containing 150 mM NaCl and 5 mM EDTA (HiLoad(TM) 16/60 Superdex(TM) 75 prep grade, available from Cytiva).

### [Example 1] Production of an scFv (Tras-scFv-HL-Glue) derived from trastuzumab

A plasmid pET28-Tras-scFv-HL-Glue was constructed by the procedure described below. Using pET28-b(+) (available from Merck Millipore) as a template, a vector fragment was amplified by PCR with the following primer set.
catggtatatctccttcttaaagttaaacaaaattatttctagaggggaattgttatccg (pET-NEBuilder-Rv-v2; SEQ ID NO: 19)
gcggccgcactcgagcaccaccacc (pET-NEBuilder-Fw; SEQ ID NO: 20)

A gene fragment encoding Tras-scFv-HL-Glue having the following nucleotide sequence was purchased from IDT (SEQ ID NO: 21).

CTTTAAGAAGGAGATATACCATG
gaagtgcagctggttgaaagcggtggcggtctggtgcaaccgggcggtagcctgcgtctgagctgcgcggcgagcggttttaacat
caaagacacctacattcactgggttcgtcaagcgccgggcaagggtctggagtgggttgcgcgtatctatccgaccaacggctacac
ccgttatgcggacagcgtgaaaggtcgttttaccattagcgcggataccagcaagaacaccgcgtacctgcagatgaacagcctgcg
tgcggaagacaccgcggtttactattgcagccgttggggcggtgacggcttctacgcgatggattattggggccaaggcaccctggtg
accgttggcccggaagaacagtatagcgcgtatgcgaccaccggcgcgaccgcgggcgcgggccagagctggtatggcgaaatc
cagatgacccagagcccgagcagcctgagcgcgagcgtgggtgaccgtgttaccattacctgccgtgcgagccaagatgtgaacac
cgcggttgcgtggtatcagcaaaagccgggcaaagcgccgaagctgctgatctacagcgcgagcttcctgtatagcggtgtgccga
gccgttttagcggcagccgtagcggcaccgacttcaccctgaccattagcagcctgcagccggaggattttgcgacctactattgcca
gcaacactataccaccccgccgacctttggtcaaggcaccaaagttgaaatcaagagcGCGGCCGCACTCGAGC

The two fragments thus obtained were linked using the NEBuilder HiFi DNA assembly (available from NEB) to obtain the plasmid pET28-Tras-scFv-HL-Glue.

The amino acid sequence of the Tras-scFv-HL-Glue (SEQ ID NO: 22)

MEVQLVESGGGLVQPGGSLRLSCAASGFNIKDTYIHWVRQAPGKGLEWVA
RIYPTNGYTRYADSVKGRFTISADTSKNTAYLQMNSLRAEDTAVYYCSRWGGDGFY
AMDYWGQGTLVTVGPEEQYSAYATTGATAGAGQSWYGEIQMTQSPSSLSASVGDR
VTITCRASQDVNTAVAWYQQKPGKAPKLLIYSASFLYSGVPSRFSGSRSGTDFTLTIS
SLQPEDFATYYCQQHYTTPPTFGQGTKVEIKSAAALEHHHHHH
(GPEEQYSAYATTGATAGAGQSWYGE is the peptide linker).

The gene encoding the underlined amino acid sequence (SEQ ID NO: 23)

atggaagtgcagctggttgaaagcggtggcggtctggtgcaaccgggcggtagcctgcgtctgagctgcgcggcgag
cggttttaacatcaaagacacctacattcactgggttcgtcaagcgccgggcaagggtctggagtgggttgcgcgtatctatccgacca
acggctacacccgttatgcggacagcgtgaaaggtcgttttaccattagcgcggataccagcaagaacaccgcgtacctgcagatga
acagcctgcgtgcggaagacaccgcggtttactattgcagccgttggggcggtgacggcttctacgcgatggattattggggccaag
gcaccctggtgaccgttggcccggaagaacagtatagcgcgtatgcgaccaccggcgcgaccgcgggcgcgggccagagctggt
atggcgaaatccagatgacccagagcccgagcagcctgagcgcgagcgtgggtgaccgtgttaccattacctgccgtgcgagccaa
gatgtgaacaccgcggttgcgtggtatcagcaaaagccgggcaaagcgccgaagctgctgatctacagcgcgagcttcctgtatagc
ggtgtgccgagccgttttagcggcagccgtagcggcaccgacttcaccctgaccattagcagcctgcagccggaggattttgcgacc
tactattgccagcaacactataccaccccgccgacctttggtcaaggcaccaaagttgaaatcaagagc

The Tras-scFv-HL-Glue was prepared as follows. SHuffle T7 *E. coli* (available from NEB) was co-transformed with the pET28-Tras-scFv-HL-Glue and the pET21-Erv1p-DsbC. Transformants were selected by spreading on LB plates containing ampicillin (100 mg/L), kanamycin (50 mg/L), and streptomycin (50 mg/L). The colony was inoculated into 50 mL of the 2x YT culture medium containing ampicillin (100 mg/L), kanamycin (50 mg/L), and streptomycin (50 mg/L), and cultured overnight at 30°C. Cells were collected by centrifugal separation (at 6,000 rpm, for 10 min., at 25°C), resuspended in 300 mL of the 2x YT culture medium containing ampicillin (100 mg/L), kanamycin (50 mg/L), and streptomycin (50 mg/L), and stirred at 30°C until the optical density at 600 nm reached 2.5 to 3.0. The Tras-scFv, the DsbC, and the Erv1p was induced with 1 mM IPTG. After stirring the culture solution at 15°C for 48 hours or more, cells were collected by centrifugal separation (at 6,000 rpm, for 10 min., at 4°C), washed with 30 mL of physiological saline, and collected again by centrifugal separation (at 6,000 rpm, for 20 min, at 4°C). The collected cells were suspended in a buffer containing 50 mM Tris-HCl (with a pH value of 8.0) and 150 mM NaCl, and disrupted by sonication on ice. After centrifugal separation (at 12,000 rpm, for 40 min., at 4°C), the supernatant was collected and loaded onto a column packed with Ni-NTA agarose (available from FUJIFILM Wako Pure Chemical). The column was washed with 10 bed volumes (20 mL) of 50 mM Tris-HCl (with a pH value of 8.0) containing both 500 mM NaCl and 20 mM imidazole. The scFv protein was eluted with a solution of 50 mM Tris-HCl (with a pH value of 8.0), 300 mM NaCl, and 250 mM imidazole. The eluate was collected and subjected to centrifugal separation (at 12,000 rpm, for 10 min., at 4°C), and then purified by gel filtration chromatography using a HiLoad 16/600 Superdex 75 pg (prep grade) column (available from GE Healthcare) with a running buffer of 50 mM HEPES (with a pH value of 7.4) containing 150 mM NaCl and 5 mM EDTA (HiLoad(TM) 16/60 Superdex(TM) 75 prep grade, available from Cytiva).

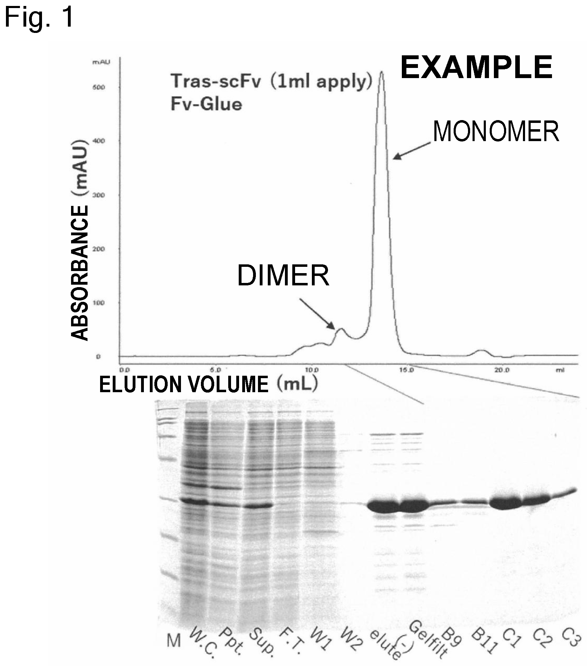

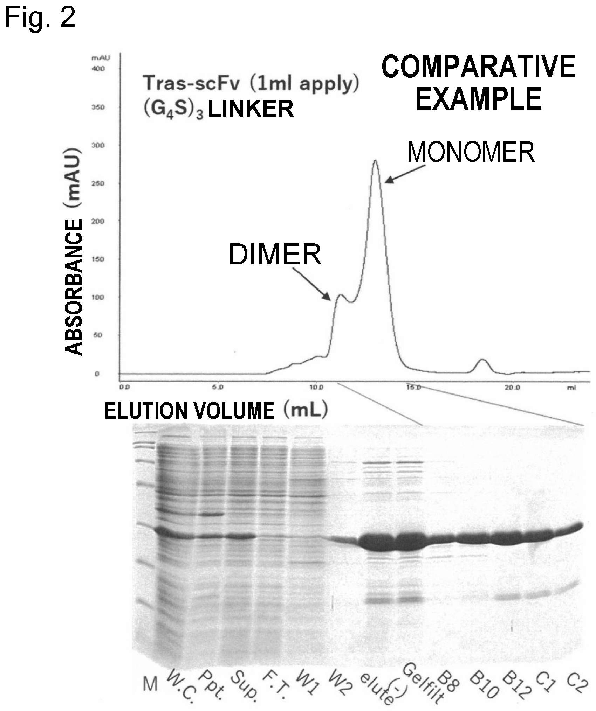

For the scFv (Tras-scFv-HL-Glue) having a peptide linker including the sequence of SEQ ID NO: 2 and an scFv having a (G₄S)₃ (SEQ ID NO: 46) linker (Tras-scFv-HL: a (G₄S)₃ linker, a control), the results of purification by gel filtration chromatography are shown in Fig. 1 and Fig. 2 (yield: 2.6 mg/300 mg medium). The conditions for the gel filtration chromatography are shown below.
System: AKTA(TM) explore (room temperature, available from Cytiva);
Column: HiLoad(TM) 16/600 Superdex(TM) 75 prep grade (available from Cytiva);
Flow rate: 1 mL/min.;
Fraction volume: 1.5 mL;
Buffer: 50 mM HEPES (with a pH value of 7.4, at 4°C), 150 mM NaCl, 5 mM EDTA;
Sample loop: 5 mL.

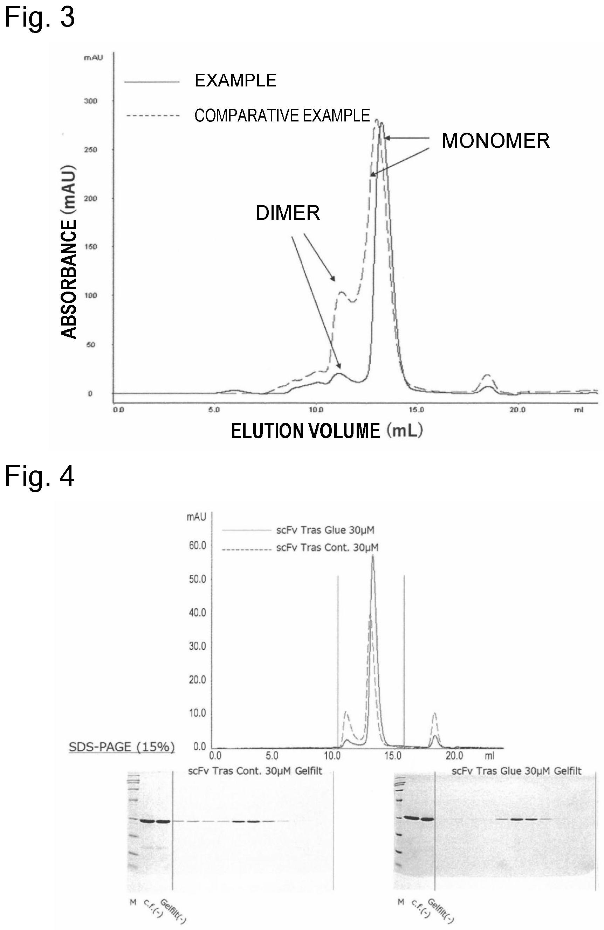

The scFv (Tras-scFv-HL-Glue) prepared as described above and the scFv comprising the (G₄S)₃ linker (Tras-scFv-HL: (G4S)₃ linker, the control) were concentrated, and analyzed by gel filtration chromatography. The results are shown in Fig. 4. The conditions for the gel filtration chromatography are shown below.
System: AKTA(TM) explore (room temperature, available from Cytiva);
Column: Superdex(TM) 75 Increase 10/300 GL (available from Cytiva);
Flow rate: 0.5 mL/min.;
Fraction volume: 0.5 mL;
Buffer: 50 mM HEPES (with a pH value of 7.4, at 4°C), 150 mM NaCl, 5 mM EDTA;
Sample loop: 0.1 mL;
Sample concentration: 30 µM.

It was confirmed that the Tras-scFv-HL-Glue had markedly reduced associativity as compared with the control (Fig. 3 and Fig. 4).

The aggregation stability of the prepared scFv was evaluated by dynamic light scattering (DLS). The DLS measurements were carried out at a wavelength of 662 nm using a DynaPro NanoStar system (available from Wyatt Technology). The molecular size distribution was analyzed with a DYNAMICS V7 software. Samples were concentrated to 3 mg/mL and incubated at 4°C. Immediately before the measurement, the samples were subjected to centrifugal separation (at 15,000 rpm, for 30 min., at 4°C) and kept on ice until the measurement. For each sample, 4 µL of solution was measured in a disposable cuvette at 25°C, and an acquisition time for each measurement was set to five seconds and the number of acquisitions was set to 10.

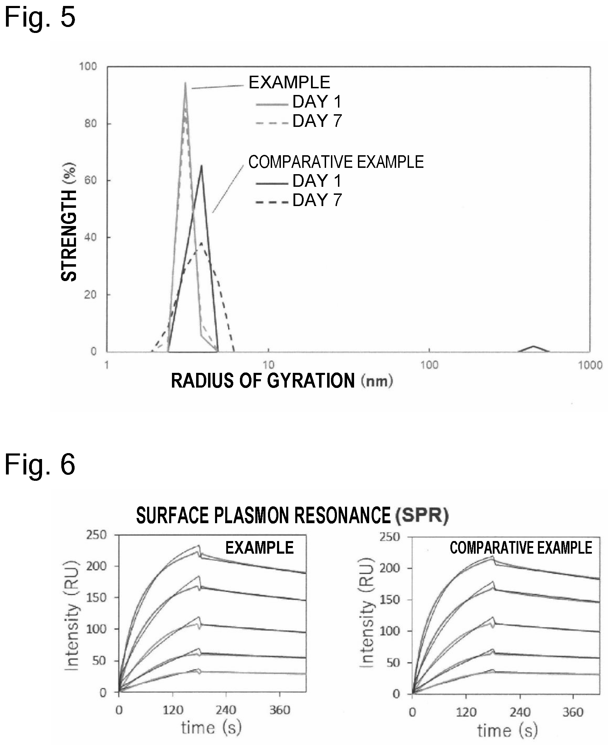

The results are shown in Fig. 5. It is suggested that, since the Tras-scFv-HL-Glue had a smaller radius of gyration than that of the control, the formation of an aggregate between the VH domain and the VL domain within the molecule was promoted. In addition, it was confirmed that in the Tras-scFv-HL-Glue the intermolecular formation of the aggregate over time was inhibited.

The antigen binding activity of the prepared scFv was evaluated by surface plasmon resonance (SPR).

Measurements were carried out using a Biacore T200 (available from Cytiva). For the measurement, a fusion protein (GST-HER2) of a peptide fragment including a trastuzumab-binding region of HER2 and glutathione S-transferase (GST) was immobilized on a sensor chip. A Series S Sensor Chip CM5 (available from Cytiva) was used as the sensor chip, and GST-HER2 was immobilized using a GST Capture Kit (available from Cytiva). Under conditions at 25°C, the Tras-scFv-HL-Glue and the control Tras-scFv-HL were prepared to 70 nM in an HBS-EP buffer, and then subjected to the measurement. The results are shown in Table 1 and Fig. 6. It was confirmed that the Tras-scFv-HL-Glue exhibited antigen binding activity equivalent to that of the control Tras-scFv-HL.

**[Table 1]**

| [Table 1] Results of Test for Confirming Antigen Bindability | | | |
|---|---|---|---|
| | *kₐ* × 10⁵(1/Ms) | *k*_{d} × 10⁻⁴(1/s) | *K*_{D} × 10⁻⁹(M) |
| Control | 4.0 ± 0.1 | 5.2 ± 0.1 | 1.3 ± 0.1 |
| Fv-Glue | 3.5 ± 0.1 | 5.4 ± 0.1 | 1.6 ± 0.1 |

Three-dimensional structural models of the scFvs (the Tras-scFv-HL-Glue and the Tras-scFv-LH-Glue) were obtained by molecular dynamics calculations. The calculation procedure is described below.

An initial model structure and initial candidate sequences of the Tras-scFv-HL-Glue were obtained using a structure prediction software Rosetta (https://www.rosettacommons.org/software), and molecular dynamics simulations were carried out using a molecular dynamics software AMBER. Based on the results, mutant structural models were created using a molecular visualization software UCSF-Chimera for residues showing large fluctuations within the linker sequence. The mutants were designed with the intention to strengthen an interaction between the Fv and the linker, or to strengthen an interaction within the linker. Molecular dynamics simulations of these structures were carried out using the AMBER, and the effects of the mutations were examined. The effects of the mutations were evaluated using RMSF (Root Mean Square Fluctuation), which is an index of deviation of a three-dimensional structure. The RMSF was calculated using cpptraj included in the AMBER. The design was carried out so that the RMSF of the linker region would be lower than that of the original sequence. The creation of the mutant structural models and the examination of the effects of the mutations were repeated multiple times, and ultimately, multiple candidate sequences including the Tras-scFv-HL-Glue shown in the Examples were obtained. Here, the term "mutation" means any of amino acid substitution, insertion, and deletion.

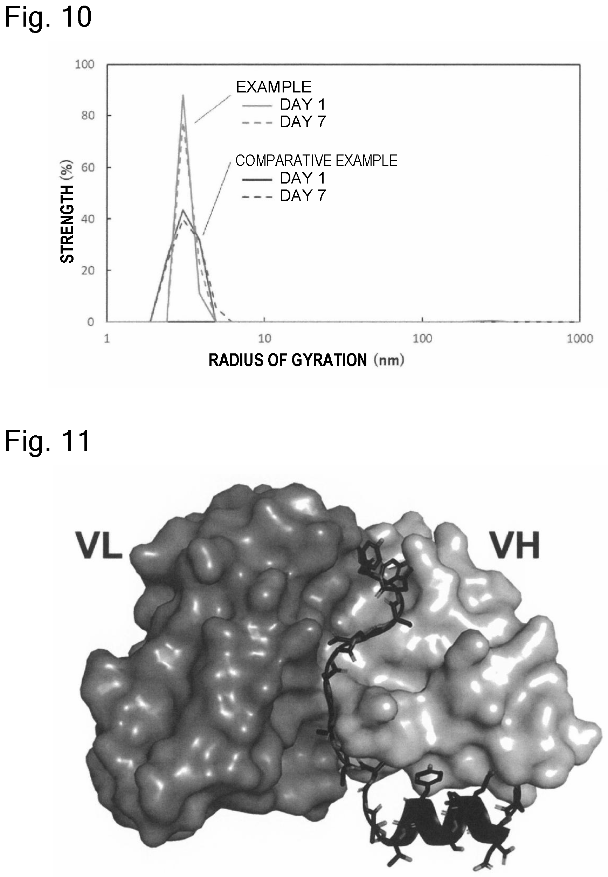

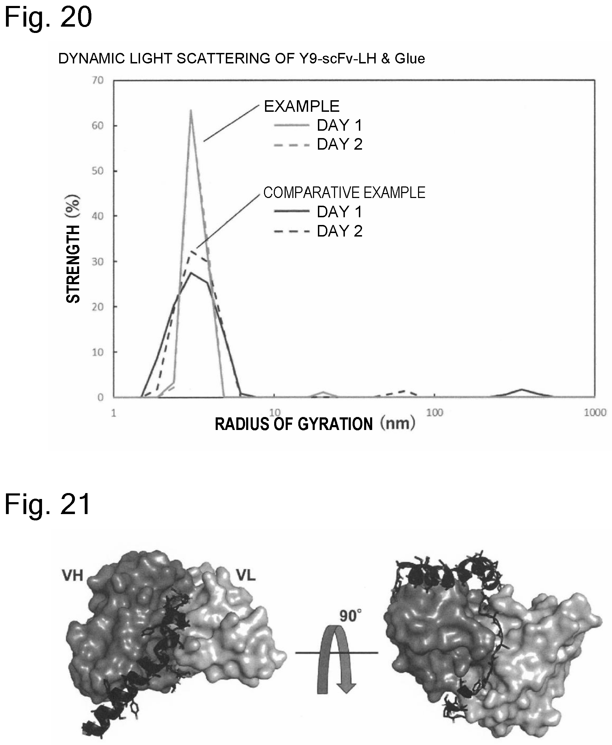

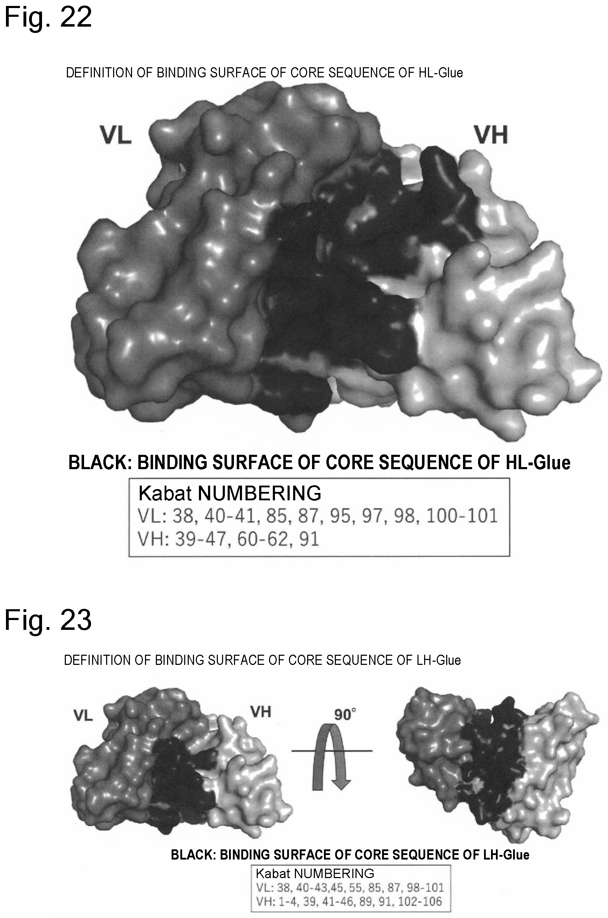

The results are shown in Figs. 11 and 21 to 23. Among the binding surfaces of the core sequence shown in Fig. 22, the following residues of the VL and the VH were identical between the Tras-scFv-HL-Glue and the Y9-scFv-HL-Glue: VL 38 (Q), 40 (P), 41 (G), 85 (T), 87 (Y), 95 (P), 97 (T), 98 (F), and 101 (G); and VH 41 (P), 42 (G), 46 (E), 62 (S), and 91 (Y), in accordance with the Kabat numbering.

Among the binding surfaces of the core sequence shown in Fig. 23, the following residues of the VL and the VH were identical between the Tras-scFv-LH-Glue and the Y9-scFv-LH-Glue: VL 38 (Q), 40 (P), 41 (G), 445 (K), 85 (T), 87 (Y), 98 (F), 99 (G), and 101 (G); and VH 2 (V), 4 (L), 41 (P), 42 (G), 43 (K), 46 (E), 91 (Y), 102 (Y), 104 (G), 105 (Q), and 106 (G), in accordance with the Kabat numbering.

### [Comparative Example 2] Production of an scFv (Y9-scFv-HL) derived from an anti-GA-pyridine antibody (Y9)

A plasmid pSAL-Y9-scFv was prepared by the method described in PTL 1 (WO 2020/013126 A1). PCR was carried out using the plasmid pSAL-Y9-scFv as a template with the following primer set to amplify a gene encoding Y9-scFv-HL.
caggggccccatatggcccaggtgaaactgcagcagtcaggacctag (Y9HL-RMH-Fw, SEQ ID NO: 24)
ctcgagtgcggccgctatttccaactttgtcccccctccgaacgtg (Y9HL-RMH-Rv, SEQ ID NO: 25)

Using pCold-MK (Kobashigawa Y., et al., Biol. Pharm. Bull. 2021; 44(1): 125-130. doi:10.1248/bpb.b20-00759) as a template, a vector fragment was amplified by PCR with the following primers (pCold-MK-NEBuilder-Fw and pCold-MK-NEBuilder-Rv).
5'-gcggccgcactcgagcaccaccacc-3' (pCold-MK-NEBuilder-Fw, SEQ ID NO: 20)
5'-catatggggcccctggaacagaacttccagcg-3' (pCold-MK-NEBuilder-Rv, SEQ ID NO: 26)

The two fragments thus obtained were linked using NEBuilder HiFi DNA assembly (available from NEB), and the resulting plasmid was named pColdMK-Y9-scFv-HL.

SHuffle T7 *E. coli* (available from NEB) was co-transformed with the pColdMK-Y9-scFv-HL and the above pET21-Erv1p-DsbC. Transformants were selected by spreading on LB plates containing ampicillin (100 mg/L), kanamycin (50 mg/L), and streptomycin (50 mg/L). The colony was inoculated into 50 mL of the 2x YT culture medium containing ampicillin (100 mg/L), kanamycin (50 mg/L), and streptomycin (50 mg/L), and cultured overnight at 30°C. Cells were collected by centrifugal separation (at 6,000 rpm, for 10 min., at 25°C), resuspended in 300 mL of the 2x YT culture medium containing ampicillin (100 mg/L), kanamycin (50 mg/L), and streptomycin (50 mg/L), and stirred at 30°C until the optical density at 600 nm reached 2.5 to 3.0. The Tras-scFv, the DsbC, and the Erv1p was induced with 1 mM IPTG. After stirring the culture solution at 15°C for 48 hours or more, cells were collected by centrifugal separation (at 6,000 rpm, for 10 min., at 4°C), washed with 30 mL of physiological saline, and collected again by centrifugal separation (at 6,000 rpm, for 20 min., at 4°C). The collected cells were suspended in a buffer containing 50 mM Tris-HCl (with a pH value of 8.0) and 150 mM NaCl and 2 mg of HRV3C protease, and disrupted by sonication on ice. After centrifugal separation (at 12,000 rpm, for 40 min., at 4°C), the supernatant was collected and loaded onto a column packed with Ni-NTA agarose (available from FUJIFILM Wako Pure Chemical). The column was washed with 10 bed volumes (20 mL) of 50 mM Tris-HCl (with a pH value of 8.0) containing both 500 mM NaCl and 20 mM imidazole. The scFv protein was eluted with a solution of 50 mM Tris-HCl (with a pH value of 8.0), 300 mM NaCl, and 250 mM imidazole. The eluate was collected and subjected to centrifugal separation (at 12,000 rpm, for 10 min., at 4°C), and then purified by gel filtration chromatography using the HiLoad 16/600 Superdex 75 pg (prep grade) column (available from GE Healthcare) with a running buffer of 50 mM HEPES (with a pH value of 7.4) containing 300 mM NaCl and 5 mM EDTA (HiLoad(TM) 16/600 Superdex(TM) 75 prep grade, available from Cytiva).
The amino acid sequence of the Y9-scFv-HL (SEQ ID NO: 27) 
GPHMAQVKLQQSGPSLVKPSQTLSLTCSVTGDSITSGYWNWIRKFPGNKFEYLGYIS
YSGRTYYNPSLKSRISITRDTSKNQYYLQLNSVTTEDTATYYCSRPYYRYDYAIDYW
GQGTTVTVSSGGGGSGGGGSGGGGSDIELTQSPAIMSASLGEQVTMTCTASSSVSSS
YLHWYQQKPGSSPKLWIYSTSNLASGVPARFSSSGSGTSYSLTISRMEAEDAATYYC
QQSWKAPYTFGGGTKLEIAAALEHHHHHH (GGGGSGGGGSGGGGS is the peptide
linker).
The gene encoding the underlined portion of the above sequence (SEQ ID NO: 28):

gcccaggtgaaactgcagcagtcaggacctagcctcgtgaaaccttctcagactctgtccctcacctgttctgtcactggcgactccatc
accagtggttactggaactggatccggaaattcccagggaataaatttgagtacttgggttacataagctacagtggtcgcacttactac
aatccatctctcaaaagtcgaatctccatcactcgagacacatccaagaaccagtactacctgcagttgaattctgtgactactgaggac
acagccacatattactgttcaagaccctactataggtacgactatgctatagactactggggccaagggaccacggtcaccgtctcctc
aggtggaggcggttcaggcggaggtggctctggcggtggcggatcggacatcgagctcactcagtctccagcaatcatgtctgcatc
tctaggggaacaggtcaccatgacctgcactgccagctcaagtgtaagttccagttacttgcactggtaccagcagaagccaggatcc
tcccccaaactctggatttatagcacatccaacctggcttctggagtcccagctcgcttcagtagcagtggatctgggacctcttactctct
cacaatcagccgaatggaggctgaagatgctgccacctattactgccagcaaagttggaaggccccgtacacgttcggagggggga
caaagttggaaata

### [Example 2] Production of an scFv (Y9-scFv-HL-Glue) derived from the anti-GA-pyridine antibody (Y9)

A plasmid pColdMK-Y9-scFv-HL-Glue was constructed by the method described below. PCR was carried out using the above plasmid pColdMK-Y9-scFv-HL as a template with the following primer set to amplify a vector fragment.
gacggtgaccgtggtcccttggccccagtagtctatagc (Y9-LinkRep-Rv, SEQ ID NO: 29)
atcgagctcactcagtctccagcaatcatgtctgcatctctagg (Y9-LinkRep-Fw, SEQ ID NO: 30)

Using the plasmid pET28-Tras-scFv-HL-Glue as a template, a gene fragment encoding the linker region was amplified by PCR with the following primer set.
gaccacggtcaccgtcggcccggaagaacagtatagcgcgtatgcg (Y9-LinkV3-Fw, SEQ ID NO: 31)
gagactgagtgagctcgatttcgccataccagctctggcccgcgcc (Y9-LinkV3-Rv, SEQ ID NO: 32)

The two fragments thus obtained were linked using the NEBuilder HiFi DNA assembly (available from NEB), and the resulting plasmid was named pColdMK-Y9-scFv-HL-Glue.

SHuffle T7 *E. coli* (available from NEB) was co-transformed with the pColdMK-Y9-scFv-HL-Glue and the above pET21-Erv1p-DsbC. Transformants were selected by spreading on LB plates containing ampicillin (100 mg/L), kanamycin (50 mg/L), and streptomycin (50 mg/L). The colony was inoculated into 50 mL of 2x YT culture medium containing ampicillin (100 mg/L), kanamycin (50 mg/L), and streptomycin (50 mg/L), and cultured overnight at 30°C. Cells were collected by centrifugal separation (at 6,000 rpm, for 10 min., at 25°C), resuspended in 300 mL of the 2x YT culture medium containing ampicillin (100 mg/L), kanamycin (50 mg/L), and streptomycin (50 mg/L), and stirred at 30°C until the optical density at 600 nm reached 2.5 to 3.0. The Tras-scFv, the DsbC, and the Erv1p was induced with 1 mM IPTG. After stirring the culture solution at 15°C for 48 hours or more, cells were collected by centrifugal separation (at 6,000 rpm, for 10 min., at 4°C), washed with 30 mL of physiological saline, and collected again by centrifugal separation (at 6,000 rpm, for 20 min., at 4°C). The collected cells were suspended in a buffer containing 50 mM Tris-HCl (with a pH value of 8.0) and 150 mM NaCl and 2 mg of HRV3C protease, and disrupted by sonication on ice. After centrifugal separation (at 12,000 rpm, for 40 min., at 4°C), the supernatant was collected and loaded onto a column packed with Ni-NTA agarose (available from FUJIFILM Wako Pure Chemical). The column was washed with 10 bed volumes (20 mL) of 50 mM Tris-HCl (with a pH value of 8.0) containing both 500 mM NaCl and 20 mM imidazole. The scFv protein was eluted with a solution of 50 mM Tris-HCl (with a pH value of 8.0), 300 mM NaCl, and 250 mM imidazole. The eluate was collected and subjected to centrifugal separation (at 12,000 rpm, for 10 min., at 4°C), and then purified by gel filtration chromatography using the HiLoad 16/600 Superdex 75 pg (prep grade) column (available from GE Healthcare) with a running buffer of 50 mM HEPES (with a pH value of 7.4) containing 300 mM NaCl and 5 mM EDTA (HiLoad(TM) 16/600 Superdex(TM) 75 prep grade, available from Cytiva).

The amino acid sequence of the Y9-scFv-HL-Glue (SEQ ID NO: 33) 
GPHMAQVKLQQSGPSLVKPSQTLSLTCSVTGDSITSGYWNWIRKFPGNKFE
YLGYISYSGRTYYNPSLKSRISITRDTSKNQYYLQLNSVTTEDTATYYCSRPYYRYDY
AIDYWGQGTTVTVGPEEQYSAYATTGATAGAGQSWYGEIELTQSPAIMSASLGEQV
TMTCTASSSVSSSYLHWYQQKPGSSPKLWIYSTSNLASGVPARFSSSGSGTSYSLTISR
MEAEDAATYYCQQSWKAPYTFGGGTKLEIAAALEHHHHHH
 (the underlined portion is the peptide linker)

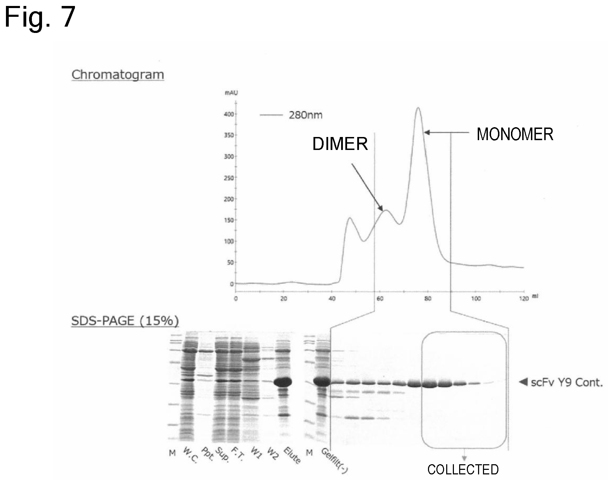

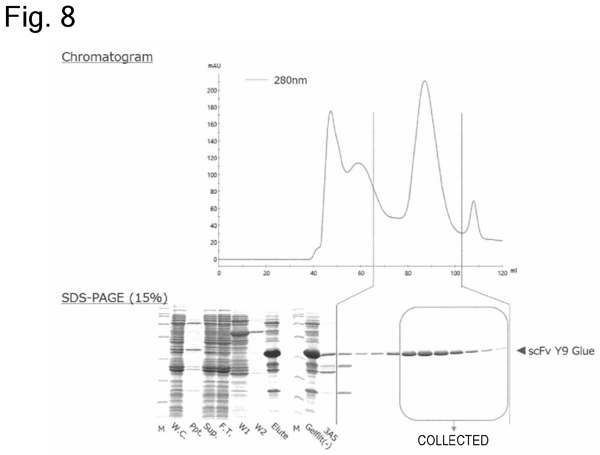

For the scFv (Y9-scFv-HL-Glue) having a peptide linker including the sequence of SEQ ID NO: 2 and an scFv having the (G₄S)₃ linker (Y9-scFv-HL: a (G₄S)₃ linker, a control), the results of analysis by gel filtration chromatography are shown in Fig. 7 and Fig. 8. The conditions for the gel filtration chromatography are shown below.
System: AKTA(TM) explore (room temperature, available from Cytiva);
Column: HiLoad(TM) 16/600 Superdex(TM) 75 prep grade (available from Cytiva);
Flow rate: 0.5 mL/min.;
Fraction volume: 0.5 mL;
Buffer: 50 mM HEPES (with a pH value of 7.4, at 4°C), 300 mM NaCl, 5 mM EDTA;
Sample loop: 5 mL.

It was confirmed that the Y9-scFv-HL-Glue had markedly reduced associativity as compared with the control (Fig. 8).

The aggregation stability of the prepared scFv was evaluated by dynamic light scattering (DLS). The DLS measurements were carried out at a wavelength of 662 nm using the DynaPro NanoStar system (available from Wyatt Technology). The molecular size distribution was analyzed with the DYNAMICS V7 software. The samples were concentrated to 3 mg/mL and incubated at 4°C. Immediately before the measurement, the samples were subjected to centrifugal separation (at 15,000 rpm, for 30 min., at 4°C) and kept on ice until the measurement. For each sample, 4 µL of the solution was measured in a disposable cuvette at 25°C, and the acquisition time for each measurement was set to five seconds and the number of acquisitions was set to 10.

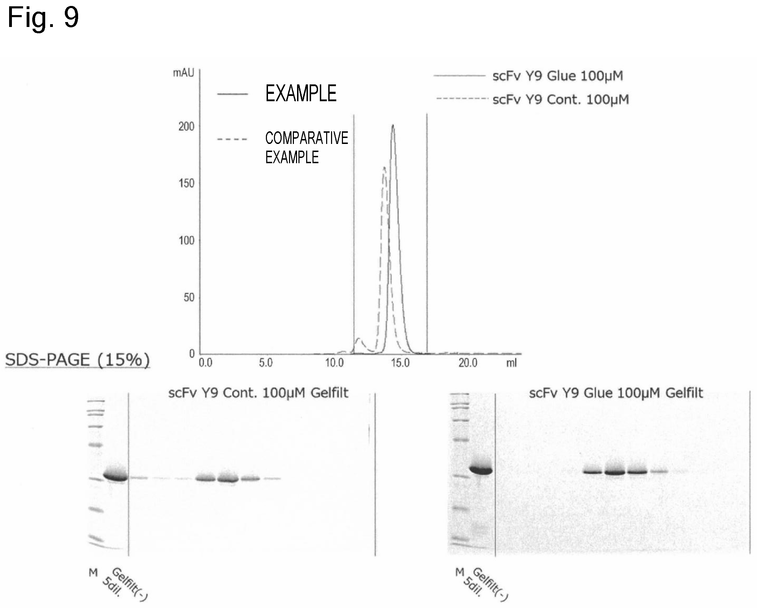

The results are shown in Fig. 9. It is suggested that, since the Y9-scFv-HL-Glue had a smaller radius of gyration than that of the control, the formation of an aggregate between the VH domain and the VL domain within the molecule was promoted. In addition, it was confirmed that in the Y9-scFv-HL-Glue the intermolecular formation of the aggregate over time was inhibited.

### [Comparative Example 3] Production of an scFv (Tras-scFv-LH) derived from trastuzumab

A plasmid pET28-Tras-scFv-LH was constructed by the method described below. Using the pET28-b(+) (available from Merck Millipore) as a template, a vector fragment was amplified by PCR with the primer set described above (the pET-NEBuilder-Rv-v2 (SEQ ID NO: 19) and the pET-NEBuilder-Fw (SEQ ID NO: 20)).

The following gene sequence encoding Tras-scFv-LH (SEQ ID NO: 34) was amplified by PCR:

CTTTAAGAAGGAGATATACCATG
Gatatccagatgacccagagcccgagcagcctgagcgcgagcgtgggtgaccgtgttaccattacctgccgtgcgagccaagatgt
gaacaccgcggttgcgtggtatcagcaaaagccgggcaaagcgccgaagctgctgatctacagcgcgagcttcctgtatagcggtgt
gccgagccgttttagcggcagccgtagcggcaccgacttcaccctgaccattagcagcctgcagccggaggattttgcgacctactat
tgccagcaacactataccaccccgccgacctttggtcaaggcaccaaagttgaaatcggtagcggtggcggaggtagcggtggtgg
cggtagtggtggtggaggttccgaagtgcagctggttgaaagcggtggcggtctggtgcaaccgggcggtagcctgcgtctgagct
gcgcggcgagcggttttaacatcaaagacacctacattcactgggttcgtcaagcgccgggcaagggtctggagtgggttgcgcgta
tctatccgaccaacggctacacccgttatgcggacagcgtgaaaggtcgttttaccattagcgcggataccagcaagaacaccgcgta
cctgcagatgaacagcctgcgtgcggaagacaccgcggtttactattgcagccgttggggcggtgacggcttctacgcgatggattat
tggggccaaggcaccctggtgaccgttagcagc
GCGGCCGCACTCGAGC

The following primer set was used for the amplification of the gene.
ctttaagaaggagatataccatggatatccagatgacccagagcccgagc (TrasLH-pET-NEB-Fw, SEQ ID NO: 35)
gctcgagtgcggccgcgctgctaacggtcaccagggtgccttggc (TrasLH-pET-NEB-Rv, SEQ ID NO: 36)

The two fragments thus obtained were linked using the NEBuilder HiFi DNA assembly (available from NEB), and the resulting plasmid was named pET28-Tras-scFv-LH. Using the plasmid pET28-Tras-scFv-LH, the Tras-scFv-LH was prepared in the same manner as in Comparative Example 1.

The amino acid sequence of the Tras-scFv-LH (SEQ ID NO: 37)

MDIQMTQSPSSLSASVGDRVTITCRASQDVNTAVAWYQQKPGKAPKLLIYSASFLYS
GVPSRFSGSRSGTDFTLTISSLQPEDFATYYCQQHYTTPPTFGQGTKVEIGSGGGGSG
GGGSGGGGSEVQLVESGGGLVQPGGSLRLSCAASGFNIKDTYIHWVRQAPGKGLEW
VARIYPTNGYTRYADSVKGRFTISADTSKNTAYLQMNSLRAEDTAVYYCSRWGGDG
FYAMDYWGQGTLVTVSSAAALEHHHHHH (the underlined portion is the peptide
linker).

### [Example 3] Production of an scFv (Tras-scFv-LH-Glue) derived from trastuzumab

A plasmid pET28-Tras-scFv-LH-Glue was constructed by the method described below. Using the pET28-b(+) (available from Merck Millipore) as a template, a vector fragment was amplified by PCR with the primer set described above (the pET-NEBuilder-Rv-v2 (SEQ ID NO: 19) and the pET-NEBuilder-Fw (SEQ ID NO: 20)).

The following gene sequence (SEQ ID NO: 38) encoding the Tras-scFv-LH-Glue was purchased from IDT.

CTTTAAGAAGGAGATATACCATG
gacattcagatgacccaaagtccatcaagtctttccgcgtcagtcggcgaccgcgttacaattacttgtcgcgcatcgcaggacgtaaa
cacggccgtggcttggtaccaacagaagcccggtaaagccccaaagctgttgatctactctgcctcattcttatatagcggagtcccttc
tcgcttctctggctcgcgcagtggtacagactttactttgacgatctctagcctgcaacctgaagacttcgcgacatactactgtcaacag
cattatactaccccacctacttttggtcagggaacgaaggtagaaatcgtagaaatcggtcccgcagctgctgccactagcacttatacg
tacgctactgccactgcgacaactggaaccacgtcctacacggctaacacaaccggacgcccgggtgctgccgcaggtggttccgg
aagcggtgctgacggcgcaacttacacgaccacgccaggtcaagtccagctggtggagtccggtggaggcctggtgcagcctggc
ggctccttacgtctgtcttgtgctgcctccggtttcaacattaaggacacctacatccactgggttcgccaggcacccggtaaaggtcttg

agtgggtggcacgtatttacccaactaatggctacactcgctacgctgacagcgtaaaagggcgctttactatctccgcagataccagc
aaaaacacggcatacctgcaaatgaattcacttcgtgctgaagacacggcagtgtattactgctcacgctggggcggagacggattct
acgcaatggactattgggggcagggcacgttggtgacagttagttcaGCGGCCGCACTCGAGC

The two fragments thus obtained were linked using the NEBuilder HiFi DNA assembly (available from NEB), and the resulting plasmid was named pET28-Tras-scFv-LH-Glue. Using the plasmid pET28-Tras-scFv-LH-Glue, the Tras-scFv-LH-Glue was prepared in the same manner as in Example 1.

In addition, for the Tras-scFv-LH and the Tras-scFv-LH-Glue, analysis by gel filtration chromatography and DLS measurements were also carried out in the same manner as in Example 1.

The amino acid sequence of the Tras-scFv-LH-Glue (SEQ ID NO: 39)

MDIQMTQSPSSLSASVGDRVTITCRASQDVNTAVAWYQQKPGKAPKLLIYSASFLYS
GVPSRFSGSRSGTDFTLTISSLQPEDFATYYCQQHYTTPPTFGQGTKVEIVEIGPAAAA
TSTYTYATATATTGTTSYTANTTGRPGAAAGGSGSGADGATYTTTPGQVQLVESGG
GLVQPGGSLRLSCAASGFNIKDTYIHWVRQAPGKGLEWVARIYPTNGYTRYADSVK
GRFTISADTSKNTAYLQMNSLRAEDTAVYYCSRWGGDGFYAMDYWGQGTLVTVSS
AAALEHHHHHH (the underlined portion is the peptide linker).

The antigen-binding activities of the Tras-scFv-LH and the Tras-scFv-LH-Glue were evaluated by a pull-down assay by the procedure described below. A buffer of 50 mM HEPES (with a pH value of 7.4), 5 mM EDTA, and 150 mM NaCl was used as a wash buffer, and a buffer of 50 mM HEPES (with a pH value of 7.4), 150 mM NaCl, 5 mM EDTA, and 30 mM reduced glutathione was used as an elution buffer.

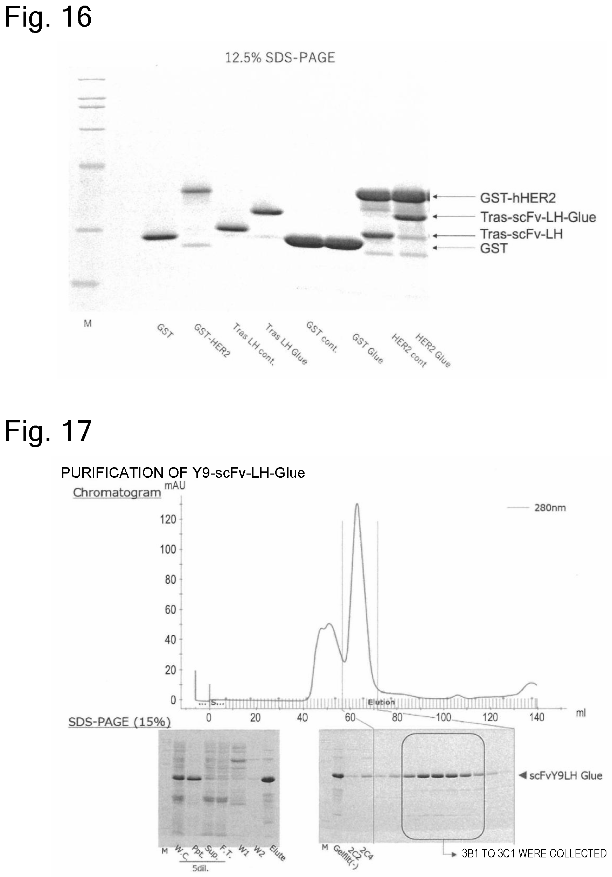

50 µL of glutathione Sepharose 4B (GS4B) resin was placed in a 1.5 mL tube, washed three times with Milli-Q water, and equilibrated three times with the wash buffer. 500 µL of GST-hHER2 (4 µM) as an antigen was added to the resin, and the mixture was stirred under rotation at 4°C for two hours. The resin was washed twice with the wash buffer, and 500 µL of the Tras-scFv-LH or the Tras-scFv-LH-Glue (4 µM) was added to the resin, followed by stirring under rotation at 4°C for two hours. After centrifugal separation (at 4°C, at 1000 rpm, for 1 min.), the supernatant fraction was removed, and the resin was transferred to a spin column and washed three times with the wash buffer. The elution buffer was added to the column, and after centrifugal separation (at 4°C, at 1000 rpm, for 1 min.), the eluted sample obtained as the supernatant fraction was subjected to 12.5% SDS-PAGE. The analysis results are shown in Fig. 16.

### [Comparative Example 4] Production of an scFv (Y9-scFv-LH) derived from the anti-GA-pyridine antibody (Y9)

A plasmid pET28-Y9-scFv-LH was constructed by the method described below. Using the pET28-b(+) (available from Merck Millipore) as a template, a vector fragment was amplified by PCR with the primer set described above (the pET-NEBuilder-Rv-v2 (SEQ ID NO: 19) and the pET-NEBuilder-Fw (SEQ ID NO: 20)).

The following gene sequence (SEQ ID NO: 40) encoding Y9-scFv-LH was purchased from IDT:

CTTTAAGAAGGAGATATACCATG
ggcagcagccatcatcatcatcatcactccagctcgctggaagttctgttccaggggccccatatggacatcgagctcactcagtctcc
agcaatcatgtctgcatctctaggggaacaggtcaccatgacctgcactgccagctcaagtgtaagttccagttacttgcactggtacca
gcagaagccaggatcctcccccaaactctggatttatagcacatccaacctggcttctggagtcccagctcgcttcagtagcagtggat
ctgggacctcttactctctcacaatcagccgaatggaggctgaagatgctgccacctattactgccagcaaagttggaaggccccgtac
acgttcggaggggggacaaagttggaaataggcagcggtggaggcggttcaggcggaggtggctctggcggtggcggatcgcag
gtgaaactgcagcagtcaggacctagcctcgtgaaaccttctcagactctgtccctcacctgttctgtcactggcgactccatcaccagt
ggttactggaactggatccggaaattcccagggaataaatttgagtacttgggttacataagctacagtggtcgcacttactacaatccat
ctctcaaaagtcgaatctccatcactcgagacacatccaagaaccagtactacctgcagttgaattctgtgactactgaggacacagcc
acatattactgttcaagaccctactataggtacgactatgctatagactactggggccaagggaccacggtcaccgtctcctcataatG
CGGCCGCACTCGAGC

The two fragments thus obtained were linked using the NEBuilder HiFi DNA assembly (available from NEB), and the resulting plasmid was named pET28-Y9-scFv-LH. Using the plasmid pET28-Y9-scFv-LH, the Y9-scFv-LH was prepared in the same manner as in Comparative Example 2.

The amino acid sequence of the Y9-scFv-LH (SEQ ID NO: 41)

MGSSHHHHHHSSSLEVLFQGPHMDIELTQSPAIMSASLGEQVTMTCTASSSV
SSSYLHWYQQKPGSSPKLWIYSTSNLASGVPARFSSSGSGTSYSLTISRMEAEDAATY
YCQQSWKAPYTFGGGTKLEIGSGGGGSGGGGSGGGGSQVKLQQSGPSLVKPSQTLS
LTCSVTGDSITSGYWNWIRKFPGNKFEYLGYISYSGRTYYNPSLKSRISITRDTSKNQY
YLQLNSVTTEDTATYYCSRPYYRYDYAIDYWGQGTTVTVSS (the underlined portion
is the peptide linker).

### [Example 4] Production of an scFv (Y9-scFv-LH-Glue) derived from the anti-GA-pyridine antibody (Y9)

A plasmid pET28-Y9-scFv-LH-Glue was constructed by the method described below. Using the pET28-b(+) (available from Merck Millipore) as a template, a vector fragment was amplified by PCR with the primer set described above (the pET-NEBuilder-Rv-v2 (SEQ ID NO: 19) and the pET-NEBuilder-Fw (SEQ ID NO: 20)).

The following gene sequence encoding Y9-scFv-LH-Glue (SEQ ID NO: 42) was purchased from IDT.

CTTTAAGAAGGAGATATACCATG
ggcagccatcaccatcaccatcattcatcatcattagaagtcttagagttcggtccacatatggctgatatcgaattaactcagtctcccgc
aatcatgtcggccagcttaggtgagcaggtaacaatgacttgtaccgcttcttcgagtgtatcgtcgagttacctgcactggtaccaaca
aaaaccaggttcttctcccaaactgtggatttattcaacctcaaaccttgcatctggcgtccccgctcgcttctcatcgtctggatcgggta
caagttattctcttacgatttctcgcatggaggcagaagacgcagctacctattattgccagcaatcttggaaggcgccttacacgttcgg
cggagggacaaaattggaaattggacccgccgctgcagcgacctcgacatacacctacgcgactgccacagcgacaacaggaaca
actagctacacggcaaatacaacaggccgcccgggagctgccgccgggggttcaggaagtggagccgacggtgcaacttatacca
ccacacctgggcaagtcaaacttcaacagagcggcccgtcattggtgaaaccctcgcagacccttagtttgacctgttcggtgaccgg
agacagcattacatctggctattggaactggattcgcaaatttcctggtaacaaattcgagtatcttggctatattagctattctggacgtac
ctactacaatccatcccttaaatctcgtatttccatcacccgcgacacttcaaaaaatcagtattatttgcaactgaattcggtcactacaga
agacacggctacctactattgttcacgtccgtattatcgctatgactatgctattgattactgggggcaggggacgaccgttactgtgtcat
cttaatGCGGCCGCACTCGAGC

The two fragments thus obtained were linked using the NEBuilder HiFi DNA assembly (available from NEB), and the resulting plasmid was named pET28-Y9-scFv-LH-Glue. Using the plasmid pET28-Y9-scFv-LH-Glue, the Y9-scFv-LH-Glue was prepared in the same manner as in Example 2.

In addition, for the Y9-scFv-LH and the Y9-scFv-LH-Glue, analysis by gel filtration chromatography and DLS measurements were also carried out in the same manner as in Example 2.

The amino acid sequence of the Y9-scFv-LH-Glue (SEQ ID NO: 43)

MGSHHHHHHSSSLEVLEFGPHMADIELTQSPAIMSASLGEQVTMTCTASSSV
SSSYLHWYQQKPGSSPKLWIYSTSNLASGVPARFSSSGSGTSYSLTISRMEAEDAATY
YCQQSWKAPYTFGGGTKLEIGPAAAATSTYTYATATATTGTTSYTANTTGRPGAAA
GGSGSGADGATYTTTPGQVKLQQSGPSLVKPSQTLSLTCSVTGDSITSGYWNWIRKF
PGNKFEYLGYISYSGRTYYNPSLKSRISITRDTSKNQYYLQLNSVTTEDTATYYCSRP
YYRYDYAIDYWGQGTTVTVSS (the underlined portion is the peptide linker).

### [Example 5] Production of an scFv (GlueCore-Tras-scFv-LH) derived from trastuzumab

A plasmid pET28-GlueCore-Tras-scFv-LH was constructed by the method described below. Using the pET28-b(+) (available from Merck Millipore) as a template, a vector fragment was amplified by PCR with the primer set described above (the pET-NEBuilder-Rv-v2 (SEQ ID NO: 19) and the pET-NEBuilder-Fw (SEQ ID NO: 20)).

The following gene sequence (SEQ ID NO: 44) encoding GlueCore-Tras-scFv-LH was purchased from IDT.

CTTTAAGAAGGAGATATACCATG

ggttcccatcatcaccaccaccactcatcctcacttgaggtactggagtttcaggggccacatatgggttccaccgccggtgcaggaca

gtcctggtatggagagatccaaatgacgcagtcgccctcgtcgctttcggcctccgtgggagaccgcgtcacaattacgtgccgcgc

gtcgcaagatgtaaacaccgccgttgcgtggtatcaacaga

aaccaggtaaagcaccgaaacttcttatttactccgccagttttttatattcgggtgtcccatcccgtttttccggctcgcgctctggcacag

acttcactctgacaatttcctccctgcagccggaagatttcgcgacttattattgccagcaacattatactaccccacctacgtttgggcaa

ggtactaaggtagaaatcggttcaggcggcggtggctcaggtggagggggttccggtgggggtggatcggaggtccagcttgtaga

aagcggtggaggtttagtacaaccaggggggtctcttcgtctttcttgcgcggcgtcgggattcaatatcaaggatacttacattcactg
ggtacgccaagcgcccgggaaggggcttgagtgggtggcacgtatctaccccactaacggatatacccgttatgccgattctgtcaaa
gggcgctttaccatcagcgcagatacgagcaaaaatacggcgtaccttcaaatgaattcgctgcgtgctgaagacacagcagtttatta
ctgttcccgctggggcggcgacggcttctacgctatggattactggggccaaggcacattggtcactgtgtcatcctaatGCGGCC
GCACTCGAGC

The two fragments thus obtained were linked using the NEBuilder HiFi DNA assembly (available from NEB), and the resulting plasmid was named pET28-GlueCore-Tras-scFv-LH. Using the plasmid pET28-GlueCore-Tras-scFv-LH, the GlueCore-Tras-scFv-LH was prepared in the same manner as in Example 1.

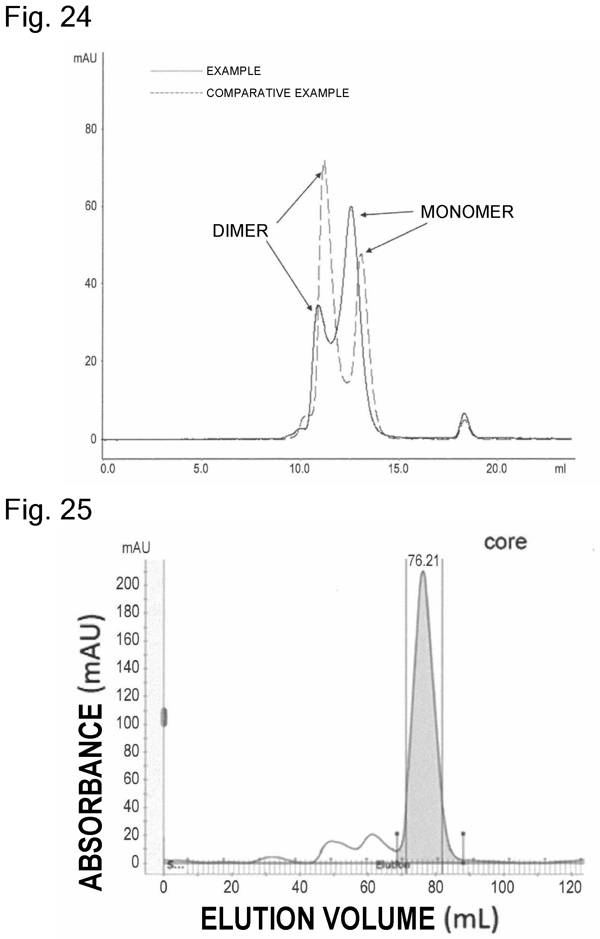

In addition, for the GlueCore-Tras-scFv-LH, analysis by gel filtration chromatography was also carried out in the same manner as in Example 1 (Fig. 24). The scFv (Tras-scFv-LH) derived from trastuzumab as prepared in [Comparative Example 3] was used as a control for comparison.

The amino acid sequence of the Tras-scFv-LH-Glue (SEQ ID NO: 45) 
MGSHHHHHH¹SSSLEVLEFQGPHMGSTAGAGQSWYG²EIQMTQSPSSLSASVGDRVT
ITCRASQDVNTAVAWYQQKPGKAPKLLIYSASFLYSGVPSRFSGSRSGTDFTLTISSL
QPEDFATYYCQQHYTTPPTFGQGTKVEIGSGGGGSGGGGSGGGGS³EVQLVESGGGL
VQPGGSLRLSCAASGFNIKDTYIHWVRQAPGKGLEWVARIYPTNGYTRYADSVKGR
FTISADTSKNTAYLQMNSLRAEDTAVYYCSRWGGDGFYAMDYWGQGTLVTVSS
 (the underlined portion ¹ is the His-tag, the underlined portion ² is the core sequence of the peptide tag, and the underlined portion ³ is the peptide linker).

### [Example 6] Production of an scFv (Tras-scFv-HL-Glue2) derived from trastuzumab

An scFv derived from trastuzumab and having the following amino acid sequence (Tras-scFv-HL-Glue2; the underlined portion is the linker) was produced by constructing the plasmid pET28-Tras-scFv-HL-Glue2 described below and carrying out the procedure described in Example 1 (yield: 3.7 mg/300 mL medium).
The amino acid sequence of Tras-scFv-HL-Glue2 (SEQ ID NO: 51) EVQLVESGGGLVQPGGSLRLSCAASGFNIKDTYIHWVRQAPGKGLEWVARIYPTNG 
YTRYADSVKGRFTISADTSKNTAYLQMNSLRAEDTAVYYCSRWGGDGFYAMDYW
GQGTLVTVGGGGSGGGGSGGGSTAGAGQSWYGEIQMTQSPSSLSASVGDRVTITCR
ASQDVNTAVAWYQQKPGKAPKLLIYSASFLYSGVPSRFSGSRSGTDFTLTISSLQPED
FATYYCQQHYTTPPTFGQGTKVEIKS
The following gene sequence encoding the Tras-scFv-HL-Glue2 (SEQ ID NO: 52)

CTTTAAGAAGGAGATATACCATG
gaagtgcagctggttgaaagcggtggcggtctggtgcaaccgggcggtagcctgcgtctgagctgcgcggcgagcggttttaacat
caaagacacctacattcactgggttcgtcaagcgccgggcaagggtctggagtgggttgcgcgtatctatccgaccaacggctacac
ccgttatgcggacagcgtgaaaggtcgttttaccattagcgcggataccagcaagaacaccgcgtacctgcagatgaacagcctgcg
tgcggaagacaccgcggtttactattgcagccgttggggcggtgacggcttctacgcgatggattattggggccaaggcaccctggtg
accgttgggggagggggttcgggtggggggggttcggggggaggcagcactgcaggagctggtcaatcctggtatggggaaatc
cagatgacccagagcccgagcagcctgagcgcgagcgtgggtgaccgtgttaccattacctgccgtgcgagccaagatgtgaacac
cgcggttgcgtggtatcagcaaaagccgggcaaagcgccgaagctgctgatctacagcgcgagcttcctgtatagcggtgtgccga
gccgttttagcggcagccgtagcggcaccgacttcaccctgaccattagcagcctgcagccggaggattttgcgacctactattgcca
gcaacactataccaccccgccgacctttggtcaaggcaccaaagttgaaatcaagagcGCGGCCGCACTCGAGC

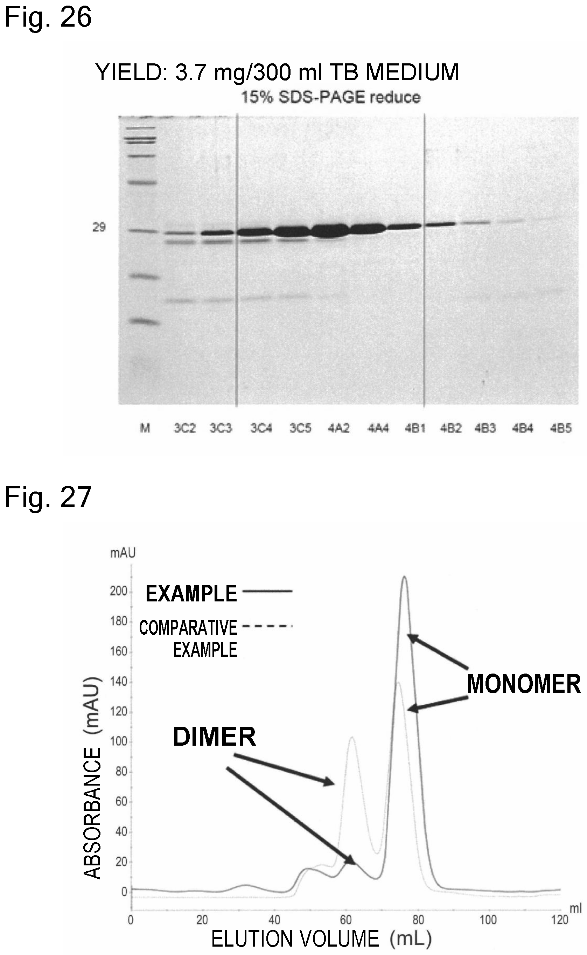

Fig. 27 shows a comparison of the results of purification by gel filtration chromatography between the scFvs (the Tras-scFv-HL and the Tras-scFv-HL-Glue2) and the control.

## Claims

1. A polypeptide comprising a heavy chain variable region (VH) domain and a light chain variable region (VL) domain that are linked via a peptide linker, wherein the peptide linker comprises an amino acid sequence:
X¹²X¹³X¹⁴GX¹⁵GQX¹⁶WX¹⁷X¹⁸ (SEQ ID NO: 1); or
AX³⁴X³⁵X³⁶SGX³⁷X³⁸X³⁹X⁴⁰X⁴¹X⁴²T (SEQ ID NO: 2), and
wherein each of X¹² to X¹⁸ and X³⁴ to X⁴² is independently selected from naturally occurring amino acid residues.

2. The polypeptide according to claim 1, wherein the peptide linker comprises an amino acid sequence:
TAGAGQSWYG (SEQ ID NO: 3); or
AAGGSGSGADGAT (SEQ ID NO: 4).

3. The polypeptide according to claim 1, wherein the peptide linker comprises an amino acid sequence:
X¹PX²X³X⁴X⁵X⁶X⁷X⁸X⁹X¹⁰X¹¹GX¹²X¹³X¹⁴GX¹⁵GQX¹⁶WX¹⁷X¹⁸ (SEQ ID NO: 5); or
X³¹X³²X³³AX³⁴X³⁵X³⁶SGX³⁷X³⁸X³⁹X⁴⁰X⁴¹X⁴²TX⁴³X⁴⁴X⁴⁵X⁴⁶X⁴⁷X⁴⁸ (SEQ ID NO: 6), and
wherein each of X¹ to X¹⁸ and X³¹ to X⁴⁸ is independently selected from naturally occurring amino acid residues.

4. The polypeptide according to claim 1, wherein the peptide linker comprises an amino acid sequence:
X¹PX²X³X⁴X⁵X⁶X⁷X⁸X⁹X¹⁰X¹¹GATAGAGQSWYG (SEQ ID NO: 7); or
X³¹X³²X³³AAGGSGSGADGATX⁴³X⁴⁴X⁴⁵X⁴⁶X⁴⁷X⁴⁸ (SEQ ID NO: 8), and
wherein each of X¹ to X¹¹, X³¹ to X³³, and X⁴³ to X⁴⁸ is independently selected from naturally occurring amino acid residues.

5. The polypeptide according to claim 1, wherein the peptide linker comprises a sequence consisting of 15 or more consecutive amino acids contained in the amino acid sequence:
GPEEQYSAYATTGATAGAGQSWYG (SEQ ID NO: 9);
PGAAAGGSGSGADGATYTTTPG (SEQ ID NO: 10); or
GGGGSGGGGSGGGSTAGAGQSWYG (SEQ ID NO: 47).

6. The polypeptide according to any one of claims 1 to 5, wherein the peptide linker consists of from 15 to 60 amino acids.

7. The polypeptide according to claim 5, wherein the peptide linker comprises a sequence consisting of 20 or more consecutive amino acids contained in the amino acid sequence of SEQ ID NO: 9 or SEQ ID NO: 10.

8. The polypeptide according to any one of claims 1 to 7, wherein the peptide linker comprises the amino acid sequence of SEQ ID NO: 9 or SEQ ID NO: 10.

9. The polypeptide according to any one of claims 1 to 8, wherein the peptide linker comprises the amino acid sequence of SEQ ID NO: 1, and is linked to a C terminus of the VH domain and an N terminus of the VL domain.

10. The polypeptide according to any one of claims 1 to 8, wherein the peptide linker comprises the amino acid sequence of SEQ ID NO: 2, and is linked to a C terminus of the VL domain and an N terminus of the VH domain.

11. The polypeptide according to claim 9, wherein the peptide linker consists of from 15 to 30 amino acids.

12. The polypeptide according to claim 10, wherein the peptide linker consists of from 35 to 60 amino acids.

13. The polypeptide according to any one of claims 1 to 12, wherein formation of an aggregate is inhibited as compared with a single-chain antibody having the same VH domain and the same VL domain, and having a peptide linker of (G₄S)₃.

14. The polypeptide according to any one of claims 1 to 13, wherein the polypeptide is a single-chain antibody (scFv).

15. A polypeptide comprising a heavy chain variable region (VH) domain and a light chain variable region (VL) domain that are linked via a linker, wherein the polypeptide has a peptide tag at an N terminus, the peptide tag comprising an amino acid sequence:
X¹²X¹³X¹⁴GX¹⁵GQX¹⁶WX¹⁷X¹⁸ (SEQ ID NO: 1); or
AX³⁴X³⁵X³⁶SGX³⁷X³⁸X³⁹X⁴⁰X⁴¹X⁴²T (SEQ ID NO: 2), and
wherein each of X¹² to X¹⁸ and X³⁴ to X⁴² is independently selected from naturally occurring amino acid residues.

16. The polypeptide according to claim 15, wherein the peptide tag comprises an amino acid sequence:
TAGAGQSWYG (SEQ ID NO: 3); or
AAGGSGSGADGAT (SEQ ID NO: 4).

17. The polypeptide according to claim 15, wherein the peptide tag comprises an amino acid sequence:
X¹PX²X³X⁴X⁵X⁶X⁷X⁸X⁹X¹⁰X¹¹GX¹²X¹³X¹⁴GX¹⁵GQX¹⁶WX¹⁷X¹⁸ (SEQ ID NO: 5); or
X³¹X³²X³³AX³⁴X³⁵X³⁶SGX³⁷X³⁸X³⁹X⁴⁰X⁴¹X⁴²TX⁴³X⁴⁴X⁴⁵X⁴⁶X⁴⁷X⁴⁸ (SEQ ID NO: 6), and
wherein each of X¹ to X¹⁸ and X³¹ to X⁴⁸ is independently selected from naturally occurring amino acid residues.

18. The polypeptide according to claim 15, wherein the peptide tag comprises an amino acid sequence:
X¹PX²X³X⁴X⁵X⁶X⁷X⁸X⁹X¹⁰X¹¹GATAGAGQSWYG (SEQ ID NO: 7); or
X³¹X³²X³³AAGGSGSGADGATX⁴³X⁴⁴X⁴⁵X⁴⁶X⁴⁷X⁴⁸ (SEQ ID NO: 8), and
wherein each of X¹ to X¹¹, X³¹ to X³³, and X⁴³ to X⁴⁸ is independently selected from naturally occurring amino acid residues.

19. The polypeptide according to claim 18, wherein the peptide tag comprises a sequence consisting of 15 or more consecutive amino acids contained in the amino acid sequence:
GPEEQYSAYATTGATAGAGQSWYG (SEQ ID NO: 9); or
PGAAAGGSGSGADGATYTTTPG (SEQ ID NO: 10).

20. The polypeptide according to claim 19, wherein the peptide tag consists of from 15 to 60 amino acids.

21. The polypeptide according to claim 19, wherein the peptide tag comprises a sequence consisting of 20 or more consecutive amino acids contained in the amino acid sequence of SEQ ID NO: 9 or SEQ ID NO: 10.

22. The polypeptide according to any one of claims 15 to 21, wherein the peptide tag comprises the amino acid sequence of SEQ ID NO: 9 or SEQ ID NO: 10.

23. The polypeptide according to any one of claims 15 to 22, wherein the peptide tag comprises the amino acid sequence of SEQ ID NO: 1, wherein the linker is linked to a C terminus of the VH domain and an N terminus of the VL domain, and wherein the peptide tag is linked to a C terminus of the VL domain.

24. The polypeptide according to any one of claims 15 to 22, wherein the peptide tag comprises the amino acid sequence of SEQ ID NO: 2, wherein the linker is linked to a C terminus of the VL domain and an N terminus of the VH domain, and wherein the peptide tag is linked to a C terminus of the VL domain.

25. The polypeptide according to claim 23, wherein the peptide tag consists of from 15 to 30 amino acids.

26. The polypeptide according to claim 24, wherein the peptide tag consists of from 35 to 60 amino acids.

27. The polypeptide according to any one of claims 15 to 26, wherein formation of an aggregate is inhibited as compared with a polypeptide having the same linker, the same VH domain, and the same VL domain, but lacking a peptide tag.

28. The polypeptide according to any one of claims 15 to 27, wherein the linker is a peptide linker.

29. The polypeptide according to any one of claims 15 to 28, wherein the polypeptide is a single-chain antibody (scFv).

30. A nucleic acid comprising a nucleotide sequence that encodes the amino acid sequence of the polypeptide according to any one of claims 1 to 29.

31. A recombinant vector comprising the nucleic acid according to claim 30.

32. A transformant into which the recombinant vector according to claim 31 has been introduced.
